(19) Europäisches Patentamt
European Patent Office
Office européen des brevets

(11) EP 1 225 172 B1

(12) # EUROPEAN PATENT SPECIFICATION

(45) Date of publication and mention of the grant of the patent:
**21.07.2004 Bulletin 2004/30**

(21) Application number: **00957113.4**

(22) Date of filing: **12.09.2000**

(51) Int Cl.[7]: **C07D 317/34**, C07D 319/06, C09D 167/00, C09J 167/00

(86) International application number:
**PCT/JP2000/006220**

(87) International publication number:
**WO 2001/021611 (29.03.2001 Gazette 2001/13)**

(54) **POLYORTHOESTER AND CURABLE COMPOSITION CONTAINING THE SAME**

POLYORTHOESTER UND HÄRTBARE ZUSAMMENSETZUNG, DIE DIESE ENTHALTEN

POLYORTHOESTER ET COMPOSITION RETICULABLE CONTENANT CE COMPOSE

(84) Designated Contracting States:
**DE GB**

(30) Priority: **17.09.1999 JP 26382099**
**22.10.1999 JP 30089499**
**29.11.1999 JP 33783799**

(43) Date of publication of application:
**24.07.2002 Bulletin 2002/30**

(73) Proprietor: **KANSAI PAINT CO., LTD.**
**Amagasaki-shi, Hyogo-ken 661-8555 (JP)**

(72) Inventors:
- **ISAKA, Hisashi**
  **Atsugi-shi, Kanagawa 243-0039 (JP)**
- **HONMA, Hiroyuki, Kansai Paint Co., Ltd.**
  **Hiratsuka-shi, Kanagawa 254-0016 (JP)**
- **MATSUNO, Yoshizumi**
  **Hadano-shi, Kanagawa 257-0004 (JP)**
- **AIDA, Haruhiko**
  **Hiratsuka-shi, Kanagawa 254-0077 (JP)**

(74) Representative: **Albrecht, Thomas, Dr. et al**
**Kraus & Weisert,**
**Thomas-Wimmer-Ring 15**
**80539 München (DE)**

(56) References cited:
**EP-A1- 0 866 065**
**WO-A1-91/03510**
**JP-A- 2000 144 040**
**US-A- 3 514 428**
**US-A- 3 515 694**
**US-A- 4 101 323**

- **DATABASE CROSSFIRE BEILSTEIN [Online] Beilstein Institut zur Förderung der Chemischen Wissenschaften, Frankfurt am Main, DE; RN 8001962, 25 January 1999 (1999-01-25) XP002219980 & CHEN ET AL.: TETRAHEDRON, vol. 54, no. 31, 1998, pages 9067-9078,**
- **DATABASE CA [Online] CHEMICAL ABSTRACTS SERVICE, COLUMBUS, OHIO, US; DN - 114:62063, XP002219981 & HOUGHTON, ROY P.; DUNLOP, JANE E.: "Enhanced reactivity of chelating ortho esters and dithio ortho esters" SYNTHETIC COMMUNICATIONS , vol. 20, no. 16, 1990, pages 2387-23400,**
- **DATABASE CROSSFIRE BEILSTEIN [Online] Beilstein Institut zur Förderung der Chemischen Wissenschaften, Frankfurt am Main, DE; RN 5171287, 1992 XP002219982 & ODINOKOV ET AL.: J. ORG. CHEM. USSR (ENGL. TRANSL.), vol. 16, 1980, pages 453-463,**
- **NARAIN R.P. & KAUR A.J.: 'Reactions of ethyl orthoacetate with glycols' INDIAN J. CHEM. vol. 17B, 1979, pages 189 - 191, XP002934790**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 225 172 B1

## Description

The present invention relates to a polyorthoester and a curable composition containing the same, more specifically to a novel polyorthoester which has a low viscosity and is readily controlled in a molecular weight and which has a high degree of freedom in molecular design and a production process for the same, and a curable composition containing the above polyorthoester, which can be reduced in a viscosity and increased in a solid content.

## Background Art

An orthoester has so far been used as a dehydrating agent and a synthetic raw material for and various compounds. Further, orthoester is known as a protective group for a hydroxyl group and protects a hydroxyl group on such moderate conditions as a room temperature in the presence of an acid catalyst by reaction as shown in the following equation (i):

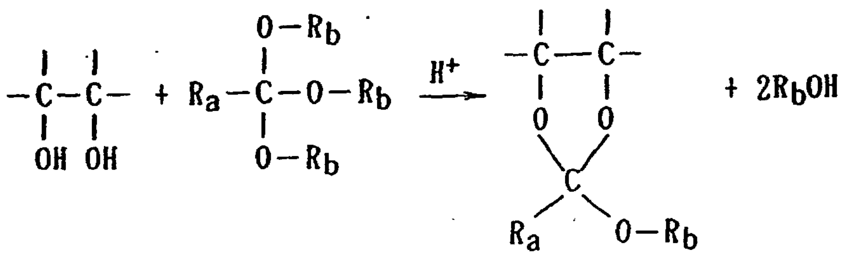
(i)

The protected hydroxyl group is stable under a base condition, but the protective group is readily desorbed under an acid condition by hydrolysis as shown in the following equation (ii) or (iii):

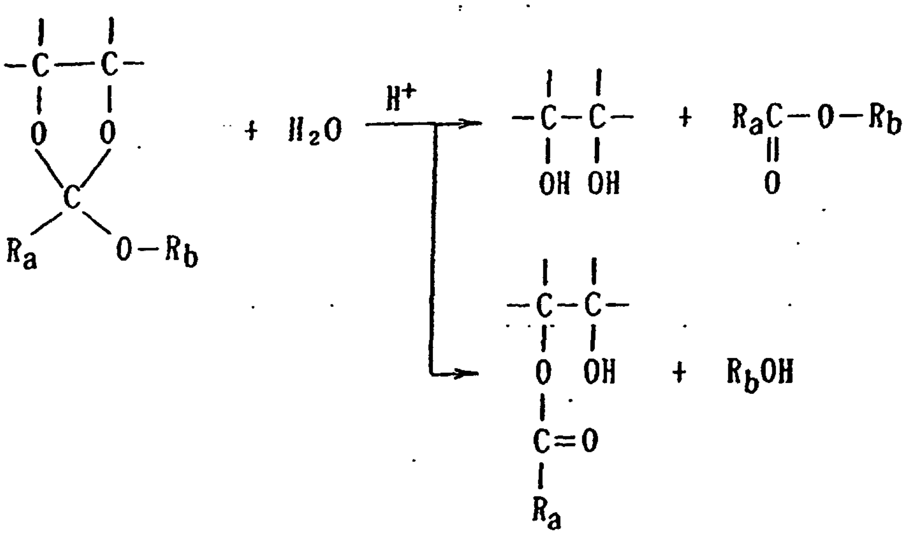
(ii)
(iii)

In general, an orthoester is easily hydrolyzed to form two molecules of alcohol and one molecule of ester:

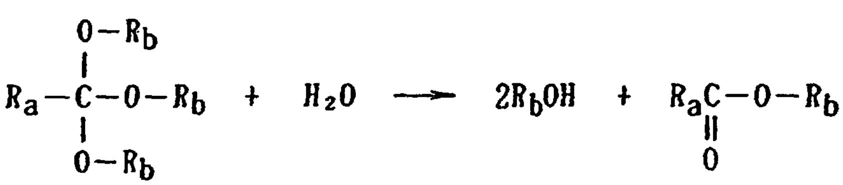
(iv)

Proposed are several techniques on a polyorthoester industrially making use of such characteristics of the polyorthoester. For example, a polyorthoester for a photoresist is described in Japanese Patent Publication No. 20325/1988 (=U.S. Patent No.4,311,782), and a polyorthoester for drug delivery is described in Japanese Patent Application Laid-Open (through PCT) No. 502465/1993 (=U.S Patent No.5,824,343).

The polyorthoesters described in these official gazettes are compounds which are obtained by condensing triols with orthoesters and which have a specific repetitive unit, for example, a repetitive unit represented by the following formula (v):

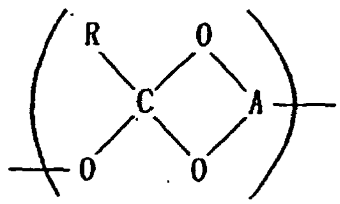

(v)

However, in producing this compound, only specific triol can be used as a hydroxy group-containing compound, and it has a degree of a freedom only in such an extent that a molecular weight can be changed by a blending ratio of the triol to the orthoester, so that there is the problem that a degree of freedom in molecular design is low.

Further, a polymer having a spiroorthoester structure is described in Japanese Patent Application Laid-Open No.42724/1982 (=U.S Patent No.4,368,314), and it is shown that the above polymer is a cross-linking high polymer having a small volumetric shrinkage. However, lactone is essentially used as a raw material, and therefore a degree of freedom in molecular design is low. Further, a polymer having a bicycloorthoester structure is described in Japanese Patent Application Laid-Open No.233114/1985, and it is shown that the above polymer is a cross-linking high polymer having an excellent balance between an elastic modulus and a toughness. However, trimethylolpropane or trimethylolethane is essentially used as a raw material, and therefore involved therein is the problem that a degree of freedom in molecular design is low.

Further, described in EP-A-882106 is a coating material composition which contains a bicyclo- or spiro-orthoester-functional compound and which has a low volatile organic substance content (VOC) and a long pot life. However, this coating material composition has to use triol, tetraol, lactone or oxetane as a raw material, and therefore involved as well therein is the problem that a degree of freedom in molecular design is low.

It is known, as described above, that an alkoxy group of an orthoester is subjected to alcohol exchange reaction with a hydroxyl group in the presence of an acid catalyst, and a 5-membered ring, a 6-membered ring or a bicyclo ring can be formed by using a hydroxyl group-containing compound in which two hydroxyl groups are close. Making use of this property, an orthoester is used as a protective group for close hydroxyl groups mainly in the biochemical field.

Three alkoxy groups of an orthoester can be subjected to alcohol exchange reaction, and it can be turned into a polyorthoester by combining with polyhydric alcohol. In this case, if an orthoester is subjected merely to exchange reaction with polyhydric alcohol (condensation reaction by dealcohol), it is gelatinized by converting into three dimension.

Further, a curable resin composition prepared by combining a polyhydric hydroxyl group-containing compound or a resin with a curing agent has so far been known, and the above curable resin composition has the problems that it is increased in a viscosity which originates in a hydroxyl group and reduced in a compatibility and that it is increased in a polarity and deteriorated in a wetting property when coated on a base material and it is inferior in storage stability when mixed with a curing agent having a reactivity with a hydroxyl group at a room temperature such as a polyisocyanate compound.

One object of the present invention is to provide a novel polyorthoester having a high degree of freedom in molecular design and a low viscosity and capable of being readily controlled in a molecular weight.

One more object of the present invention is to provide an industrially useful process in which capable of being easily produced is a novel polyorthoester having a high degree of freedom in molecular design and a low viscosity and capable of being readily controlled in a molecular weight.

A different object of the present invention is to provide a curable composition using a polyorthoester having a high degree of freedom in molecular design and a low viscosity and capable of being readily controlled in a molecular weight, in which there involved is no problem originating in a hydroxyl group, for example, a problem on an increase in a viscosity of the curable composition; the solid content can be elevated; and the compatibility, the wetting property to a base material and the storage stability are good.

Further different object of the present invention is to provide a curable composition using a polyorthoester having a high degree of freedom in molecular design and a low viscosity and capable of being readily controlled in a molecular weight, in which there involved are no problems on a curability of the curable composition and a reduction in the hardness; the viscosity can be reduced, and the solid content can be elevated; and the compatibility and the wetting property to a base material are good.

The other objects and the characteristics of the present invention shall be apparent from the following descriptions.

**Disclosure of the Invention**

Thus, the present invention provides a polyorthoester prepared by reacting:

(a) an orthoester represented by the following Formula (I):

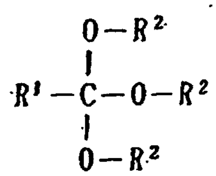 (I)

wherein $R^1$ represents a hydrogen atom or an alkyl group having 1 to 4 carbon atoms, and three $R^{2'}$s may be the same or different and each represent an alkyl group having 1 to 4 carbon atoms,
(b) at least one glycol compound selected from α-glycols and β-glycols, and
(c) a hydroxyl group-containing compound having at least two hydroxyl groups in a molecule other than the compound (b) described above.

Further, the present invention provides a production process for a polyorthoester characterized by subjecting the orthoester (a), the glycol compound (b) and the hydroxyl group-containing compound (c) each described above to condensation reaction in the presence of an acid catalyst.

Further, the present invention provides a curable composition (hereinafter referred to as a curable composition I) comprising:

(A) the polyorthoester described above, and
(B) a curing agent having a group having a reactivity with a hydroxyl group.

Further, the present invention provides a curable composition (hereinafter referred to as a curable composition II) comprising:

(1) a base polymer having a reactivity with the following curing agent (2),
(2) a curing agent having a reactivity with a hydroxyl group and having a reactivity with the base polymer (1), and
(3) the polyorthoester described above.

The polyorthoester and the curable composition of the present invention shall be explained below in further details.

<u>Polyorthoester</u>

The polyorthoester of the present invention is a reaction product of the orthoester (a), the glycol compound (b) and the hydroxyl group-containing compound (c) each described below.

<u>Orthoester (a)</u>:

The orthoester which is the component (a) is a compound represented by the following Formula (I):

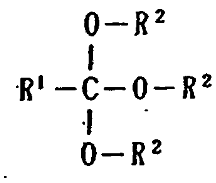

(I)

wherein $R^1$ represents a hydrogen atom or an alkyl group having 1 to 4 carbon atoms, and three $R^{2'}$s may be the same or different and each represent an alkyl group having 1 to 4 carbon atoms.

In Formula (I) described above, the "alkyl group having 1 to 4 carbon atoms" represented by $R^1$ or $R^2$ is linear or branched and includes, for example, methyl, ethyl, n-propyl, isopropyl, n-butyl, isobutyl and t-butyl.

The specific examples of the orthoester (a) include, for example, methyl orthoformate, ethyl orthoformate, propyl orthoformate, butyl orthoformate, methyl orthoacetate, ethyl orthoacetate, methyl orthopropionate, ethyl orthopropionate, methyl orthobutyrate and ethyl orthobutyrate. Among them, methyl orthoformate, ethyl orthoformate, methyl orthoacetate and ethyl orthoacetate are suited. They may be used alone or in combination of two or more kinds thereof.

Glycol compound (b):

The glycol compound which is the component (b) is at least one glycol compound selected from α-glycols and β-glycols having two hydroxyl groups in a molecule.

Among them, a compound represented by the following Formula (II) can suitably be used as the α-glycol:

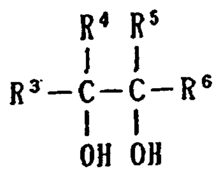

(II)

wherein $R^3$, $R^4$, $R^5$ and $R^6$ may be the same or different and each represent a hydrogen atom, an alkyl group having 1 to 24, preferably 1 to 10 carbon atoms, an aralkyl group having 7 to 24, preferably 7 to 10 carbon atoms or a phenyl group, or a group obtained by substituting a part of these groups with an oxygen atom, and the total of the carbon atoms in the groups represented by $R^3$, $R^4$, $R^5$ and $R^6$ falls in a range of 0 to 24, preferably 0 to 10; and $R^4$ and $R^5$ may form a cyclic structure together with carbon atoms to which they are bonded directly.

In Formula (II) described above, the "alkyl group having 1 to 24 carbon atoms" represented by $R^3$, $R^4$, $R^5$ or $R^6$ is linear, branched or cyclic and includes methyl, ethyl, n-propyl, isopropyl, n-butyl, isobutyl, t-butyl, pentyl, hexyl, n-octyl, 2-ethylhexyl, decyl, dodecyl, octadecyl, cyclohexyl, methylcyclohexyl, cyclohexylmethyl and cyclohexylethyl.

In Formula (II) described above, the "aralkyl group having 7 to 24 carbon atoms" represented by $R^3$, $R^4$, $R^5$ or $R^6$ is preferably an alkyl group substituted with phenyl, and the specific examples thereof include benzyl and phenethyl.

In Formula (II) described above, the "group obtained by substituting a part of the alkyl group, the aralkyl group or the phenyl group with an oxygen atom" represented by $R^3$, $R^4$, $R^5$ or $R^6$ includes, for example, an alkoxyalkyl group such as methoxymethyl, ethoxymethyl, propoxymethyl and butoxymethyl; an alkanoyloxyalkyl group such as acetoxymethyl and acetoxyethyl; and an aryloxyalkyl group such as phenoxymethyl and phenoxyethyl.

Among them, $R^3$, $R^4$, $R^5$ or $R^6$ in Formula (II) described above is preferably a hydrogen atom or an alkyl group having 1 to 6 carbon atoms.

In Formula (II) described above, the cyclic structure which can be formed by $R^4$ and $R^5$ together with carbon atoms to which they are bonded directly includes, for example, a cyclohexyl group and a cyclopentyl group.

Thus, the representative examples of the α-glycols include, for example, ethylene glycol, 1,2-propylene glycol, 1,2-butylene glycol, 2,3-butylene glycol, 1,2-hexanediol, 1,2-dihydroxycyclohexane, pinacol and hydrolysis products of long chain alkyl monoepoxides; fatty acid monoglycerides (a products) such as glycerin monoacetate (α product) and glycerin monostearate (α product); 3-ethoxypropane-1,2-diol and 3-phenoxypropane-1,2-diol. Among them, ethylene glycol, 1,2-propylene glycol and 1,2-hexanediol are suited.

On the other hand, particularly a compound represented by the following Formula (III) can suitably be used as the β-glycol:

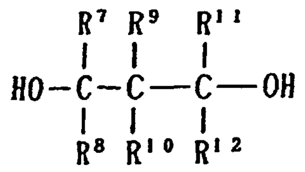 (III)

wherein $R^7$, $R^8$, $R^9$, $R^{10}$, $R^{11}$ and $R^{12}$ may be the same or different and each represent a hydrogen atom, an alkyl group having 1 to 24, preferably 1 to 10 carbon atoms, an aralkyl group having 7 to 24, preferably 7 to 10 carbon atoms or a phenyl group, or a group obtained by substituting a part of these groups with an oxygen atom, and the total of the carbon atoms in the groups represented by $R^7$, $R^8$, $R^9$, $R^{10}$, $R^{11}$ and $R^{12}$ falls in a range of 0 to 24, preferably 0 to 10; and $R^7$ and $R^9$ or $R^7$, $R^9$ and $R^{11}$ may form a cyclic structure together with carbon atoms to which they are bonded directly.

In Formula (III) described above, the "alkyl group having 1 to 24 carbon atoms" represented by $R^7$, $R^8$, $R^9$, $R^{10}$, $R^{11}$ or $R^{12}$ includes the same ones as those described above as the alkyl group represented by $R^3$, $R^4$, $R^5$ or $R^6$ in Formula (II) described above.

In Formula (III) described above, the "aralkyl group having 7 to 24 carbon atoms" represented by $R^7$, $R^8$, R9, $R^{10}$, $R^{11}$ or $R^{12}$ is preferably an alkyl group substituted with phenyl, and the specific examples thereof include benzyl and phenethyl.

In Formula (III) described above, the "group obtained by substituting a part of the alkyl group, the aralkyl group or the phenyl group with an oxygen atom" represented by $R^7$, $R^8$, $R^9$, $R^{10}$, $R^{11}$ or $R^{12}$ includes, for example, an alkoxyalkyl group such as methoxymethyl, ethoxymethyl, propoxymethyl and butoxymethyl; an alkanoyloxyalkyl group such as acetoxymethyl and acetoxyethyl; and an aryloxyalkyl group such as phenoxymethyl and phenoxyethyl.

In Formula (III) described above, the cyclic structure which can be formed by $R^7$ and $R^9$ or $R^7$, $R^9$ and $R^{11}$ together with carbon atoms to which they are bonded directly includes, for example, a cyclohexyl group and a cyclopentyl group.

Among them, $R^7$, $R^8$, $R^9$, $R^{10}$, $R^{11}$ or $R^{12}$ in Formula (III) described above is preferably a hydrogen atom or an alkyl group having 1 to 6 carbon atoms.

Thus, the representative examples of the β-glycols include, for example, neopentyl glycol, 2-methyl-1,3-propanediol, 2-methyl-2,4-pentanediol, 3-methyl-1,3-butanediol, 2-ethyl-1,3-hexanediol, 2,2-diethyl-1,3-propanediol, 2,2,4-trimethyl-1,3-pentanediol, 2-butyl-2-ethyl-1,3-propanediol, 2-phenoxypropane-1,3-diol, 2-methyl-2-phenylpropane-1,3-diol, 1,3-propylene glycol, 1,3-butylene glycol, dimethylolpropionic acid, dimethylolbutanoic acid, 2-ethyl-1,3-octanediol and 1,3-dihydroxycyclohexane; fatty acid monoglycerides (β products) such as glycerin monoacetate (β product) and glycerin monostearate (β product). Among them, suited are neopentyl glycol, 2-methyl-1,3-propanediol, 2-methyl-2,4-pentanediol, 3-methyl-1,3-butanediol, 2-ethyl-1,3-hexanediol, 2,2-diethyl-1,3-propanediol, 2,2,4-trimethyl-1,3-pentanediol and 2-butyl-2-ethyl-1,3-propanediol.

(c) Hydroxyl group-containing compound

The hydroxyl group-containing compound which is the component (c) is a compound having two or more hydroxyl groups in a molecule other than the glycol compound (b) described above.

The hydroxyl group-containing compound (c) includes compounds having two hydroxyl groups other than α-glycols and β-glycols or compounds having 3 or more, preferably 3 to 40 hydroxyl groups in a molecule.

The compounds having two hydroxyl groups other than α-glycols and β-glycols include, for example, 1,4-butanediol, 1,4-dihydroxycyclohexane, 1,5-pentanediol, 1,6-hexanediol, 2,5-hexanediol, 3-methyl-1,5-pentanediol, 1,4-dimethylolcyclohexane, tricyclodecanedimethanol, 2,2-dimethyl-3-hydroxypropyl-2,2-dimethyl-3-hydroxypropionate (this compound corresponds to ester of hydroxypivalic acid and neopentyl glycol), bisphenol A, bisphenol F, bis (4-hydroxyhexyl)-2,2-propane, bis(4-hydroxyhexyl)methane, 3,9-bis(1,1-dimethyl-2-hydroxyethyl)-2,4,8,10-tetraoxaspiro[5,5]undecane, diethylene glycol, triethylene glycol, tetra- or more polyethylene glycol, dipropylene glycol, tripropylene glycol, tetra- or more polypropylene glycol, copolymers having hydroxyl groups at both terminals obtained by copolymerizing ethylene oxide with propylene oxide, linear polyesters having hydroxyl groups at both terminals such as polycaprolactonediol, polycarbonatediol and carboxylic acid adducts of diepoxide.

The compounds described above having 3 or more hydroxyl groups include, for example, glycerin, diglycerin, triglycerin, pentaerythritol, dipentaerythritol, sorbitol, mannit, trimethylolethane, trimethylolpropane, ditrimethylolpropane, tris(2-hydroxyethyl)isocyanurate, gluconic acid and polymers having 3 or more hydroxyl groups (polyesters, polyethers, acryl polymers, ketone resins, phenol resins, epoxy resins, urethane resins those having 3 or more hydroxyl groups, polyvinyl alcohols which are saponified products of polyvinyl acetates and natural saccharides such as glucose), each of which has 3 or more hydroxyl groups.

Capable of being suitably used as the hydroxyl group-containing compound (c) are the compounds having a molecular weight falling in a range of 90 to 100,000, particularly 100 to 50,000 and further particularly 100 to 10,000 and a hydroxyl group value falling in a range of 20 to 1,850 mg KOH/g, particularly 40 to 1,650 mg KOH/g and further particularly 80 to 1,650 mg KOH/g.

Production of polyorthoester

In producing the polyorthoester of the present invention, a blending ratio of the orthoester (a), the glycol compound (b) and the hydroxyl group-containing compound (c) shall not specifically be restricted. In general, it is suitable in terms of an easiness in controlling the molecular weight to use the orthoester (a) in a proportion falling in a range of 0.01 to 10 moles, preferably 0.05 to 5 moles and more preferably 0.1 to 2 moles and the glycol compound (b) in a proportion falling in a range of 0.01 to 10 moles, preferably 0.05 to 5 moles and more preferably 0.1 to 2 moles each per equivalent of a hydroxyl group contained in the hydroxyl group-containing compound (c).

The polyorthoester of the present invention can be obtained by subjecting the three components (a), (b) and (c) described above to condensation reaction. For example, it can suitably be produced by heating the three components described above at a temperature falling in a range of usually a room temperature to 250°C, preferably 70 to 200°C for 1 to 20 hours, if necessary, in the presence of an organic solvent and an acid catalyst to subject them to condensation reaction.

In the present invention, an alkoxy group of the orthoester (a) causes exchange reaction with the alcohol parts of the glycol compound (b) and the hydroxyl group-containing compound (c). In this case, the orthoester (a) is preferentially reacted with the α-glycol or β-glycol which is the glycol compound (b) having hydroxyl groups close to each other to form a cyclic structure. That is, the glycol compound (b) is cyclized by preferentially reacting with two functional groups (alkoxyl groups) of the orthoester (a) which is trifunctional. The remaining one alkoxyl group of the orthoester is reacted with the hydroxyl group-containing compound (c). Thus, cross-linking between the molecules is not brought about in producing the polyorthoester of the present invention, and therefore the resulting polyorthoester can be inhibited to a large extent from being increased in a molecular weight and a viscosity. On the other hand, if the orthoester (a) is reacted directly with the hydroxyl group-containing compound (c) in the absence of the glycol compound (b), cross-linking between the molecules takes place, so that the product is rapidly increased in a molecular weight and a viscosity. The present invention has made it possible to inhibit the product from being increased in a molecular weight and a viscosity to control them by adding the glycol compound (b) to the orthoester (a) and the hydroxyl group-containing compound (c) to react them.

Thus, obtained is the polyorthoester having a structure in which a hydroxyl group contained in the hydroxyl group-containing compound (c) is blocked by the orthoester of a 5-membered ring or a 6-membered ring constituted by the orthoester (a) and the glycol compound (b).

When used as the raw materials are, for example, the orthoester represented by Formula (I) described above, the α-glycol represented by Formula (II) described above and the compound having 2 to 6 hydroxyl groups in a molecule, the polyorthoester of the present invention produced in the manner described above can have a structure represented by the following Formula (IV):

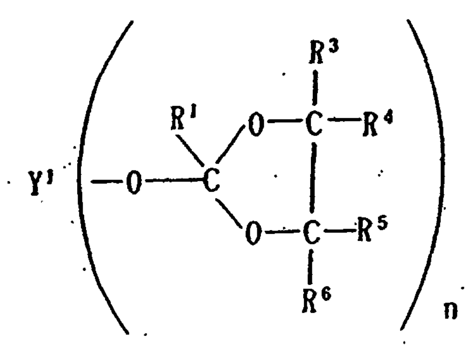

(IV)

wherein $Y^1$ represents a di- to hexavalent residue obtained by removing the following 2 to 6 hydroxyl groups from the compound having 2 to 6 hydroxyl groups in a molecule; $R^1$, $R^3$, $R^4$, $R^5$ and $R^6$ are the same as defined above; and n represents an integer of 2 to 6. Further, when used as the raw materials are, for example, the orthoester represented by Formula (I) described above, the β-glycol represented by Formula (III) described above and the compound having 2 to 6 hydroxyl groups in a molecule, the polyorthoester of the present invention produced in the manner described

above can have a structure represented by the following Formula (V):

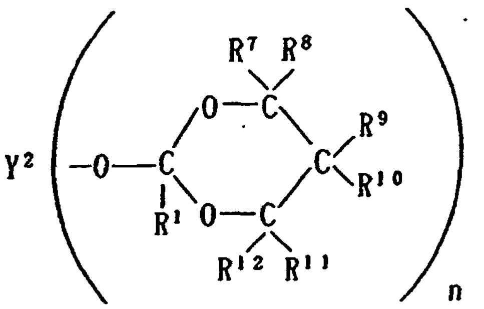

(V)

wherein $Y^2$ represents a di- to hexavalent residue obtained by removing the following 2 to 6 hydroxyl groups from the compound having 2 to 6 hydroxyl groups in a molecule; $R^1$, $R^7$, $R^8$, $R^9$, $R^{10}$, $R^{11}$ and $R^{12}$ are the same as defined above; and n represents an integer of 2 to 6.

Further, when used as the raw materials are, for example, the orthoester represented by Formula (I) described above, the α-glycol represented by Formula (II) described above and the compound having 2 hydroxyl groups in a molecule, the polyorthoester of the present invention produced in the manner described above can have a structure represented by the following Formula (VI):

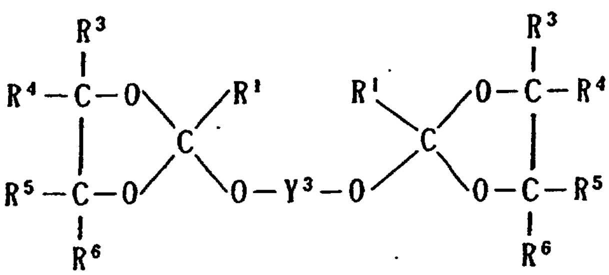

(VI)

wherein $Y^3$ represents a residue obtained by removing 2 hydroxyl groups from the compound having 2 hydroxyl groups in a molecule; and $R^1$, $R^3$, $R^4$, $R^5$ and $R^6$ are the same as defined above. Also, when used as the raw materials are, for example, the orthoester represented by Formula (I) described above, the β-glycol represented by Formula (III) described above and the compound having 4 hydroxyl groups in a molecule, the polyorthoester of the present invention produced in the manner described above can have a structure represented by the following Formula (VII):

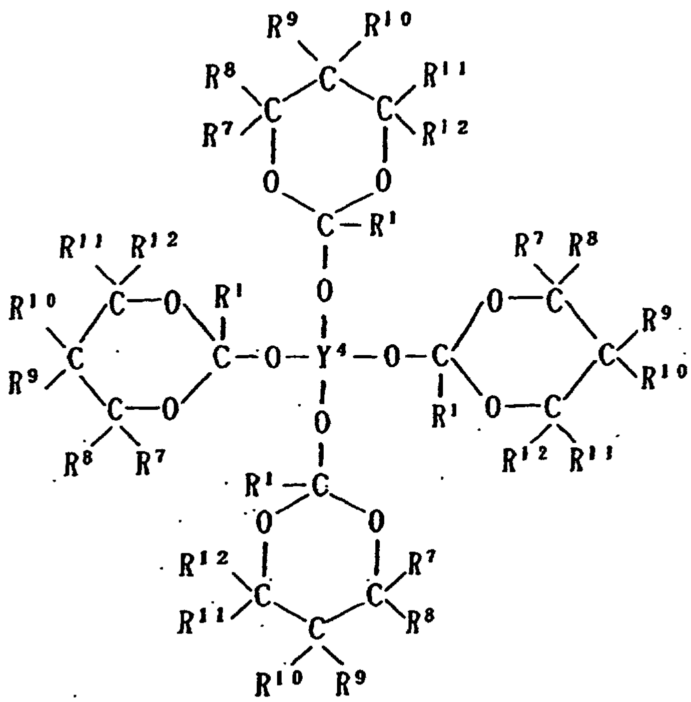

(VII)

wherein $Y^4$ represents a residue obtained by removing the four hydroxyl groups from the compound having four hydroxyl groups in a molecule; and $R^1$, $R^7$, $R^8$, $R^9$, $R^{10}$, $R^{11}$ and $R^{12}$ are the same as defined above.

Compounds of the invention have a structure represented by the Formula (VII) above or by the following Formulas (IV)' and (V)':

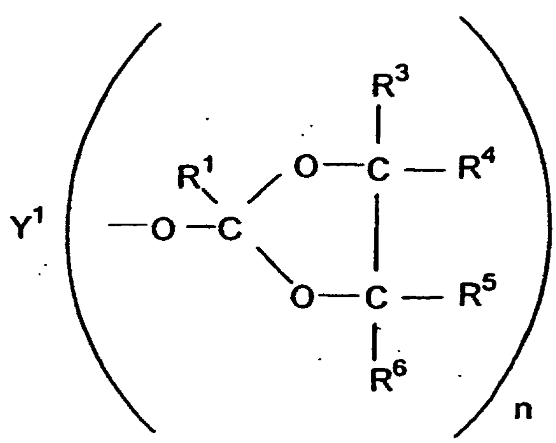

(IV)'

wherein $Y^1$ represents a residue obtained by removing 3 to 40 hydroxyl groups from a compound having 3 to 40 hydroxyl groups in a molecule; $R^1$, $R^3$, $R^4$, $R^5$ and $R^6$ are the same as defined in claims 1 and 4; and n represents an integer of 3 to 40, or

the following Formula (V):

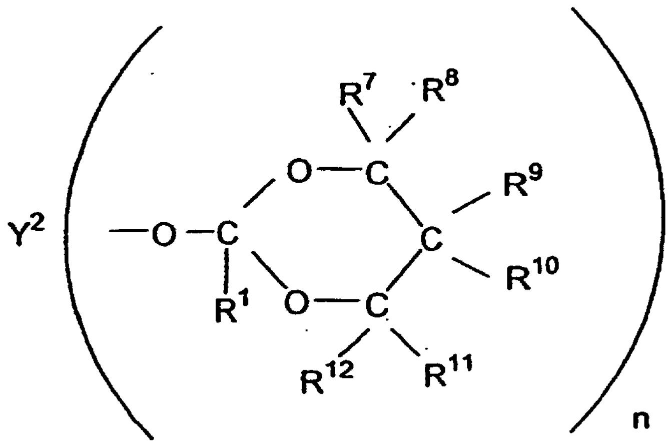

(V)'

wherein $Y^2$ represents a residue obtained by removing 3 to 40 hydroxyl groups from a compound having 3 to 40 hydroxyl groups in a molecule; $R^1$, $R^7$, $R^8$, $R^9$, $R^{10}$, $R^{11}$ and $R^{12}$ are the same as defined in claims 1 and 4; and n represents an integer of 3 to 40.

The polyorthoester provided by the present invention can be used as a binder, a cross-linking agent and a reactive diluent for coating materials, inks and adhesives. Further, it can be used as a hydrolytic polymer, a biodegradable polymer and a controlled release chemical.

Curable composition I:

The curable composition I provided by the present invention comprises:

(A) the polyorthoester of the present invention described above, and
(B) a curing agent having a group having a reactivity with a hydroxyl group.

Curing agent (B):

In this curable composition I, a compound (hereinafter, referred to as a "compound (B)") having at least two groups having a reactivity with a hydroxyl group in a molecule is used as the curing agent (B) in order to cause a three-dimensional cross-linking reaction with the polyorthoester (A), and the above compound is reacted with a hydroxyl group formed by hydrolysis of an orthoester group in the polyorthoester (A) to form a cured matter.

Capable of being given as the representative examples of the compound (B) are, for example, polyisocyanate compounds, amino resins, epoxy group-containing compounds, alkoxysilyl group-containing compounds and compounds having two or more carboxylic anhydride groups.

The polyisocyanate compounds described above include all of compounds in which isocyanato groups (NCO group) are not blocked (hereinafter abbreviated as "non-blocked polyisocyanate compound") and compounds in which isocyanato groups are partially or completely blocked (hereinafter abbreviated as "blocked polyisocyanate compound").

The non-blocked polyisocyanate compounds include, for example, aliphatic diisocyanates such as lysine diisocyanate, hexamethylene diisocyanate and trimethylhexane diisocyanate; alicyclic diisocyanates such as hydrogenated xylilene diisocyanate, isophorone diisocyanate, methylcyclohexane-2,4 or 2,6-diisocyanate, 4,4'-methylenebis (cyclohexylisocyanate) and 1,3-(isocyanatomethyl)cyclohexane; aromatic diisocyanates such as tolylene diisocyanate, xylylene diisocyanate and diphenylmethane diisocyanate; organic polyisocyanates including trivalent or more polyisocyanates such as lysine triisocyanate, adducts of these organic polyisocyanates to polyhydric alcohols, low molecular weight polyester resins or water, cyclic polymers (for example, isocyanurate) of the respective organic diisocyanates themselves described above, and biuret type adducts thereof; and copolymers of isocyanate group-containing ethylenically unsaturated compounds such as isocyanatoethyl (meth)acrylate and m-isopropenyl-α,α-dimethylbenzylisocyanate with other ethylenically unsaturated compounds.

The blocked polyisocyanate compounds are obtained by blocking the isocyanate groups of the non-blocked polyisocyanate compounds described above with a blocking agent. Capable of being suitably used as the blocking agent described above are, for example, blocking agents including phenol bases such as phenol, cresol and xylenol; lactam bases such as ε-caprolactam, δ-valerolactam, γ-butyrolactam and β-propiolactam; alcohol bases such as methanol, ethanol, n- or i-propyl alcohol, n-, i- or t-butyl alcohol, ethylene glycol monomethyl ether, ethylene glycol monoethyl ether, ethylene glycol monobutyl ether, diethylene glycol monomethyl ether, diethylene glycol monoethyl ether, propylene glycol monomethyl ether and benzyl alcohol; oxime bases such as formamidoxime, acetaldoxime, acetoxime, methyl ethyl ketoxime, diacetylmonoxime, benzophenoneoxime and cyclohexaneoxime; and active methylene bases such as dimethyl malonate, diethyl malonate, ethyl acetoacetate, methyl acetoacetate and acetylacetone. The isocyanate groups of the polyisocyanates can readily be blocked by mixing the non-blocked polyisocyanate compounds described above with the blocking agents described above.

These polyisocyanate compounds can be used alone or in combination of two or more kinds thereof.

The amino resins which can be used as the compound (B) include, for example, methylol amino resins obtained by reacting aldehydes with amino components such as melamine, urea, benzoguanamine, acetoguanamine, steroguanamine, spiroguanamine and dicyanediamide. The aldehydes described above include, for example, formaldehyde, paraformaldehyde, acetaldehyde and benzaldehyde. Further, compounds obtained by etherifying these methylol amino resins with at least one alcohol are included in the amino resins described above. The alcohols used for the etherification include, for example, monohydric alcohols such as methyl alcohol, ethyl alcohol, n-propyl alcohol, isopropyl alcohol, n-butyl alcohol, isobutyl alcohol, 2-ethylbutanol and 2-ethylhexanol. Among them, particularly suited are the melamine resins obtained by etherifying at least a part of the methylol groups of the methylol melamine resins with the monohydric alcohols having 1 to 4 carbon atoms.

Capable of being given as the specific examples of the melamine resins described above are, for example, methyl-etherified melamine resins such as Cymel 300, ditto 303, ditto 325, ditto 327, ditto 350, ditto 730, ditto 736 and ditto 738 (all described above are manufactured by Mitsui Cytec Co., Ltd.), Melan 522 and ditto 523 (all described above are manufactured by Hitachi Chemical Co., Ltd.), Nikalac MS 001, ditto MX430 and ditto MX650 (all described above are manufactured by Sanwa Chemical Co., Ltd.), Sumimal M-550, ditto M-100 and ditto M-40S (all described above are manufactured by Sumitomo Chemical Ind. Co., Ltd.) and Resimine 740 and ditto 747 (all described above are manufactured by Monsanto Co., Ltd.), butyl-etherified melamine resins such as U-van 20SE and ditto 225 (all described above are manufactured by Mitsui Toatsu Co., Ltd.) and Super Beckamine J820-60, ditto L-117-60, ditto L-109-65, ditto 47-508-60, ditto L-118-60 and ditto G821-60 (all described above are manufactured by Dainippon Ink & Chemicals Ind. Co., Ltd.); and methyl ether and butyl ether-mixed etherified melamine resins such as Cymel 232, ditto 266, ditto XV-514 and ditto 1130 (all described above are manufactured by Mitsui Cytec Co., Ltd.), Nikalac MX500, ditto MX600 ditto MS35 and ditto MS 95 (all described above are manufactured by Sanwa Chemical Co., Ltd.), Resimine 753 and ditto 755 (all described above are manufactured by Monsanto Co., Ltd.) and Sumimal M-66B (manufactured by Sumitomo Chemical Ind. Co., Ltd.). These melamine resins can be used alone or in combination of two or more kinds thereof.

The epoxy group-containing compound which can be used as the compound (B) is a compound having two or more epoxy groups in a molecule, and the representative examples thereof include ethylene glycol diglycidyl ether, polyethylene glycol diglycidyl ether, propylene glycol diglycidyl ether, tripropylene glycol diglycidyl ether, polypropylene glycol diglycidyl ether, 1,4-butanediol diglycidyl ether, neopentyl glycol diglycidyl ether, 1,6-hexanediol diglycidyl ether, glycerin diglycidyl ether, diglycerin tetraglycidyl ether, trimethylolpropane triglycidyl ether, 2,6-diglycidyl phenyl ether, sorbitol triglycidyl ether, triglycidyl isocyanurate, diglycidylamine, diglycidylbenzylamine, diglycidyl phthalate, bisphenol A diglycidyl ether, butadiene dioxide, cyclopentadiene dioxide, diester of 3,4-epoxycyclohexenecarboxylic acid and ethylene glycol, 3,4-epoxyclohexylmethyl-3,4-epoxyclohexanecarboxylate, 3,4-epoxy-6-methylclohexylmethyl-3,4-epoxy-6-methylclohexanecarboxylate, bis(3,4-epoxycyclohexylmethyl) adipate, glycidyl ether of dicyclopentadieneol epoxide, dipentene dioxide, adducts of bisphenol A type epoxy resins and ethylene oxide, Epoleade GT300 (trifunctional alicyclic epoxy compound, manufactured by Daicel Chemical Industries Ltd.), Epoleade GT400 (tetrafunctional alicyclic epoxy compound, manufactured by Daicel Chemical Industries Ltd.); Epoleade GT301, ditto GT302 and ditto GT303 (ring-opened ε-caprolactone chain-containing trifunctional alicyclic epoxy compounds, all described above are manufactured by Daicel Chemical Industries Ltd.); Epoleade GT401, ditto GT402 and ditto GT403 (ring-opened ε-caprolactone chain-containing tetrafunctional alicyclic epoxy compounds, all described above are manufactured by Daicel Chemical Industries Ltd.); Epikote 828, ditto 834 and ditto 100 (bisphenol A type epoxy resins, all described above are manufactured by Yuka Shell Epoxy Co., Ltd.); Epikote 154 (cresol novolak type epoxy resin, manufactured by Yuka Shell Epoxy Co., Ltd.); Celloxide 2081, ditto 2082 and ditto 2083 each represented by the following Formula (VIII) (all described above are manufactured by Daicel Chemical Industries Ltd.; in the following Formula (VIII), the product in which n is 1 is Celloxide 2081; the product in which n is 2 is Celloxide 2082; and the product in which n is 3 is Celloxide 2083); and Denacol EX-411 represented by the following Formula (IX) (manufactured by Nagase Kasei Co., Ltd.):

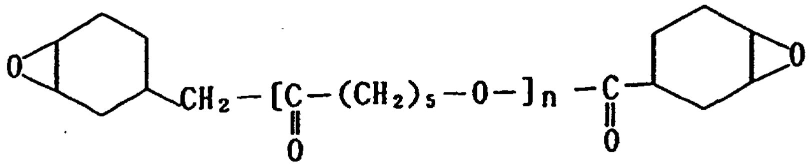 (VIII)

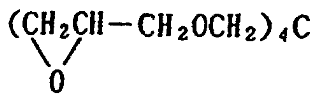 (IX)

In Formula (VIII), n represents an integer of 1 to 3.

The epoxy group-containing compound described above includes copolymers of epoxy group-containing polymerizable unsaturated monomers such as glycidyl (meth)acrylate, allyl glycidyl ether and 3,4-epoxycyclohexylmethyl (meth)acrylate with other polymerizable unsaturated monomers. The other polymerizable unsaturated monomers described above include , for example, $C_1$ to $C_{24}$ alkyl (meth)acrylates such as methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, 2-ethylhexyl acrylate, n-octyl (meth)acrylate, lauryl (meth)acrylate, tridecyl (meth)acrylate and stearyl (meth)acrylate; monoesters of polyhydric alcohols and acrylic acid or methacrylic acid such as 2-hydroxyethyl (meth) acrylate, hydroxypropyl (meth)acrylate, 2,3-dihydroxybutyl (meth)acrylate, 4-hydroxybutyl (meth)acrylate and polyethylene glycol mono(meth)acrylate; compounds obtained by subjecting ε-caprolactone to ring opening reaction with the monoesters of the polyhydric alcohols described above and acrylic acid or methacrylic acid; alkoxysilyl group-containing polymerizable unsaturated compounds such as vinyltrimethoxysilane, vinyltriethoxysilane, vinylmethyldimethoxysilane, vinylmethyldiethoxysilane, γ-(meth)acryloyloxypropyltrimethoxysilane, γ-(meth)acryloyloxypropylmethyldimethoxysilane, γ-(meth)acryloyloxypropylmethyldiethoxysilane, γ-(meth)acryloyloxypropyltriethoxysilane, β-(meth)acryloyloxyethyltrimethoxysilane and γ-(meth)acryloyloxybutylphenyldimethoxysilane; vinyl aromatic compounds such as styrene, vinyltoluene and α-methylstyrene; acrylonitrile, methacrylonitrile, tricyclodecanyl (meth)acrylate, isobornyl (meth) acrylate, vinyl acetate and Beova Monomer (manufactured by Shell Chemical Co., Ltd.).

The epoxy group-containing compounds described above can be used alone or in combination of two or more kinds thereof. A content of the epoxy group of the epoxy group-containing compound described above shall not specifically be restricted, and the epoxy equivalent falls suitably in a range of usually 100 to 3,000, preferably 100 to 500.

The alkoxysilyl group-containing compound which can be used as the compound (B) is a compound having two or more alkoxysilyl groups in a molecule and includes, for example, alkoxysilanes having no polymerizable unsaturated groups such as dimethoxydimethylsilane, dimethoxydiethylsilane, dimethoxydiphenylsilane, diethoxydimethylsilane, trimethoxymethylsilane, trimethoxyethylsilane, trimethoxypropylsilane, trimethoxyphenylsilane, tetramethoxysilane, tetraethoxysilane, tetrabutoxysilane and dimethoxydiethoxysilane; polymerizable unsaturated group-containing alkoxysilanes such as vinyltrimethoxysilane, vinyltriethoxysilane, vinylmethyldimethoxysilane, vinylmethyldiethoxysilane, γ-(meth)acryloyloxypropyltrimethoxysilane, γ-(meth)acryloyloxypropylmethyldimethoxysilane, γ-(meth) acryloyloxypropylmethyldiethoxysilane, γ-(meth)acryloyloxypropyltriethoxysilane, γ-(meth)acryloyloxyethyltrimethoxysilane and γ-(meth)acryloyloxybutylphenyldimethoxysilane; partially condensed products of the alkoxysilanes described above having no polymerizable unsaturated groups and/or the polymerizable unsaturated group-containing alkoxysilanes described above; and copolymers of the polymerizable unsaturated group-containing alkoxysilanes described above with polymerizable unsaturated monomers which can be copolymerized with the above alkoxysilanes.

The polymerizable unsaturated monomers capable of being copolymerized with the alkoxysilanes which are the monomer components for the copolymers described above include monomers other than the alkoxysilyl group-containing compounds capable of being used as the "other polymerizable unsaturated monomers" which are copolymerized with the epoxy group-containing polymerizable unsaturated monomers when the epoxy group-containing compounds described above are copolymers.

Capable of being given as the compound (hereinafter, abbreviated as a "polyacid anhydride") having two or more carboxylic anhydride groups which can be used as the curing agent (B) are, for example, pyromellitic anhydride, a condensation product [ethylenebis(anhydrotrimellitate)] of one mole of ethylene glycol and 2 moles of trimellitic anhydride, a condensation product [glycerintris-(anhydrotrimellitate)] of one mole of glycerin and 3 moles of trimellitic anhydride; linear or cyclic polyacid anhydrides obtained by subjecting polybasic acids such as succinic acid, adipic acid, azelaic acid, sebacic acid, dodecanedioic acid, dimer acid, ethyl-octadecanedioic acid, phenyl-hexadecanedioic acid and 1,4-cyclohexanedicarboxylic acid to intermolecular condensation; and polymers comprising a polymerizable

unsaturated acid anhydride such as maleic anhydride and tetrahydrophthalic anhydride as a single monomer component. The monomers other than the polymerizable unsaturated acid anhydrides which can form the above polymers include , for example, alkyl (meth)acrylates having 1 to 24 carbon atoms such as methyl (meth)acrylate, ethyl (meth) acrylate, n-propyl (meth)acrylate, isopropyl (meth)acrylate, n-butyl (meth)acrylate, isobutyl (meth)acrylate, tert-butyl (meth)acrylate, 2-ethylhexyl acrylate, n-octyl (meth)acrylate, lauryl (meth)acrylate, tridecyl (meth)acrylate and stearyl (meth)acrylate; vinyl aromatic compounds such as styrene, vinyltoluene and $\alpha$-methylstyrene; polymerizable unsaturated monocarboxylic acids such as acrylic acid, methacrylic acid and crotonic acid; acrylonitrile, methacrylonitrile, tricyclodecanyl (meth)acrylate, isobornyl (meth)acrylate, vinyl acetate and Veova Monomer (manufactured by Shell Chemical Co., Ltd.).

A content of the acid anhydride groups in the polyacid anhydrides described above shall not specifically be restricted, and the whole acid value based on the acid anhydride groups falls suitably in a range of usually 50 to 1,100 mg KOH/g, preferably 80 to 800 mg KOH/g.

Among the compounds (B) described above, the polyisocyanate compounds and the amino resins can be given as the particularly suitable compound.

The compounds (B) described above can be used alone or in combination of two or more kinds thereof.

Curable composition:

The curable composition I of the present invention comprises the polyorthoester (A) and the compound (B) each described above as the essential components. A blending proportion of these both components shall not specifically be restricted and falls suitably in a range of usually 95/5 to 20/80, preferably 90/10 to 30/70 and more preferably 70/30 to 30/70.

In addition to the polyorthoester (A) and the compound (B), the curable composition I of the present invention can suitably contain, if necessary, an acid catalyst, an organic solvent, a curing catalyst, a pigment, a UV absorber, a coated surface-controlling agent, an antioxidant, a fluidity-controlling agent and a wax.

The acid catalyst described above is a catalyst for accelerating a reaction of deblocking an orthoester group to reproduce a hydroxyl group. The kind thereof shall not specifically be restricted, and capable of being given are, for example, inorganic acids such as hydrochloric acid, sulfuric acid and nitric acid; sulfonic acid compounds such as methanesulfonic acid, ethanesulfonic acid, paratoluenesulfonic acid, dodecylbenzenesulfonic acid, dinonylnaphthalenesulfonic acid and dinonylnaphthalenedisulfonic acid; compounds obtained by neutralizing the sulfonic acid compounds described above with bases such as amines; esters of the sulfonic acid compounds described above with primary, secondary or tertiary alcohols such as n-propanol, n-butanol, n-hexanol, n-octanol, isopropanol, 2-butanol, 2-hexanol, 2-octanol, cyclohexanol and tert-butanol; $\beta$-hydroxyalkylsulfonic acid esters obtained by reacting the sulfonic acid compounds described above with oxirane group-containing compounds such as glycidyl acetate and butyl glycidyl ether; carboxylic acids such as formic acid, acetic acid, propionic acid, butyric acid, 2-ethylhexanoic acid and octanoic acid; organic phosphoric acid base compounds such as monobutyl phosphate, dibutyl phosphate, monoisopropyl phosphate, diisopropyl phosphate, monooctyl phosphate, dioctyl phosphate, monodecyl phosphate, didecyl phosphate, metaphosphoric acid, orthophosphoric acid, pyrophosphoric acid, trimethyl phosphate, triethyl phosphate, tributyl phosphate, trioctyl phosphate, tributoxyethyl phosphate, tris chloroethyl phosphate, triphenyl phosphate and tricresyl phosphate; compounds obtained by neutralizing the organic phosphoric acid base compounds described above with bases such as amines; light latent acid-generating agents which generate acids by irradiating with UV rays such as Cyracure UVI-6970, ditto UVI-6974 and ditto UVI-6990 (all described above are manufactured by Union Carbide Co., Ltd.), Irgacure 261 and ditto 264 (all described above are manufactured by Ciba Specialty Chemicals Co., Ltd.), CIT-1682 (manufactured by Nippon Soda Co., Ltd.), BBI-102 (manufactured by Midori Kagaku Co., Ltd.) and Adeka Optomer SP-150 and ditto SP-170 (all described above are manufactured by Asahi Denka Kogyo K.K.); and Lewis acids. Among them, particularly suited are the sulfonic acids compounds, the neutralized compounds of the sulfonic acids compounds, the organic phosphoric acid base compounds and the neutralized compounds of the organic phosphoric acid base compounds.

The curable composition I of the present invention can usually be a composition of a non-solvent or organic solvent type. When it is the composition of an organic solvent type, solvents which can dissolve or disperse the respective components of the curable composition I can be used, and capable of being given are, for example, hydrocarbon base solvents such as heptane, toluene, xylene, octane and mineral spirits; ester base solvents such as ethyl acetate, n-butyl acetate, isobutyl acetate, ethylene glycol monomethyl ether acetate and diethylene glycol monobutyl ether acetate; ketone base solvents such as methyl ethyl ketone, methyl isobutyl ketone, diisobutyl ketone and cyclohexanone; alcohol base solvents such as methanol, ethanol, isopropanol, n-butanol, sec-butanol and isobutanol; ether base solvents such as n-butyl ether, dioxane, ethylene glycol monomethyl ether and ethylene glycol monoethyl ether; and aromatic petroleum base solvents such as Swasol 310, Swasol 1000 and Swasol 1500 (all described above are manufactured by Cosmo Oil Co., Ltd.) and Shellsol A (manufactured by Shell Chemical Co., Ltd.). These organic

solvents can be used alone or in combination of two or more kinds thereof.

The curing catalyst described above which is blended, if necessary, with the curable composition I is intended for accelerating the curing reaction of the curable composition I, and suited when the compound (B) is a blocked polyisocyanate compound is, for example, a curing catalyst which promotes the dissociation of a blocking agent in the blocked polyisocyanate compound which is a curing agent. Capable of being given as the suited curing catalyst are, for example, organic metal catalysts such as tin octanoate, dibutyltin di(2-ethylhexanoate), dioctyltin di(2-ethylhexanoate), dioctyltin diacetate, dibutyltin dilaurate, dibutyltin oxide, dioctyltin oxide and lead 2-ethylhexanoate.

When the compound (B) is an amino resin such as a melamine resin, particularly a methyl-etherified or methyl ether and butyl ether-mixed etherified melamine resin having a low molecular weight, phosphoric acid, a sulfonic acid compound or an amine-neutralized product of the sulfonic acid compound is suitably used as the curing catalyst. The representative examples of the sulfonic acid compound include p-toluenesulfonic acid, dodecylbenzenesulfonic acid, dinonylnaphthalenesulfonic acid and dinonylnaphthalenedisulfonic acid. The amine in the amine-neutralized product of the sulfonic acid compound may be any one of primary amines, secondary amines and tertiary amines.

The curing catalyst used when the compound (B) is the epoxy group-containing compound includes, for example, chelating compounds such as tetrakis(acetylacetonato)zirconium, cobalt acetylacetonate, tris(acetylacetonato) aluminum and manganese acetylacetonate; chelating reaction products of compounds having a β-hydroxyamino structure with lead (II) oxide; metal carboxylates such as lead 2-ethylhexanoate, lead secanoate, lead naphthenoate, lead octanoate, lead acetate, lead lactate and zirconium octanoate; and imidazole compounds such as imidazole, 2-methylimidazole, 2-isopropylimidazole, 2-undecylimidazole and 2-phenylimidazole.

The curing catalyst used when the compound (B) is the alkoxysilyl group-containing compound includes, for example, organic sulfonic acid compounds such as dodecylbenzenesulfonic acid, paratoluenesulfonic acid, dinonylnaphthalenesulfonic acid and trifluorosulfonic acid; amine-neutralized products of these organic sulfonic acid compounds; and phosphoric acid base compounds such as monobutyl phosphate, dibutyl phosphate, monoisopropyl phosphate, diisopropyl phosphate, monooctyl phosphate, dioctyl phosphate, monodecyl phosphate, didecyl phosphate, metaphosphoric acid, orthophosphoric acid, pyrophosphoric acid, trimethyl phosphate, triethyl phosphate, tributyl phosphate, trioctyl phosphate, tributoxyethyl phosphate, tris chloroethyl phosphate, triphenyl phosphate and tricresyl phosphate.

The curing catalyst used when the compound (B) is the polyacid anhydride includes, for example, quaternary salt catalysts such as tetraethylammonium bromide, tetrabutylammonium bromide, tetraethylammonium chloride, tetrabutylammonium fluoride, tetrabutylphosphonium bromide, triphenylbenzylphosphonium chloride and n-dodecyltributylammonium bromide; and amines such as triethylamine and tributylamine.

Capable of being given as the pigment compounded, if necessary, with the curable composition I of the present invention are, for example, inorganic color pigments such as titanium white, carbon black, red iron oxide and titanium yellow; organic color pigments such as quinacridone red, azo red, phthalocyanine blue, phthalocyanine green and organic yellow pigments; brilliant pigments such as aluminum powder and brilliant mica powder; extender pigments such as silica powder, calcium carbonate, barium sulfate, mica, clay and talc; and rust preventive pigments such as calcium ion-exchanged silica, phosphate base rust preventive pigments and chromate base pigments.

The curable composition I of the present invention can suitably be used for uses in coating material compositions, adhesives and inks, particularly as a coating material composition. The curing conditions of the curable composition I of the present invention shall not specifically be restricted. Usually, when the curing time is 5 minutes or longer, the curing temperature falls suitably in a range of a room temperature (about 0°C) to about 200°C, particularly about 60°C to about 180°C, and when the curing time is shorter than 5 minutes, the curing temperature falls suitably in a range of about 60°C to about 300°C, particularly about 80°C to about 260°C.

<u>Curable composition II</u>:

The curable composition II of the present invention comprises a combination of

(1) a base polymer having a reactivity with the following curing agent (2),

(2) a curing agent which has a group having a reactivity with a hydroxyl group and which has a reactivity with the base polymer (1), and

(3) the polyorthoester described above according to the present invention, and it is characterized by that the viscosity can be reduced and the solid content can be elevated without bringing about problems on a curing property of the composition and a reduction in the hardness and that the compatibility and the wetting property to a base material are good.

Base polymer (1):

The base polymer (1) which is the component (1) in the curable composition II of the present invention is a polymer having a reactivity with the curing agent (2) described later. The base polymer (1) shall not specifically be restricted in a kind thereof as long as it has a reactive group (for example, a hydroxyl group, an epoxy group, a carboxyl group, an alkoxysilyl group, an acid anhydride group and an isocyanate group) capable of being reacted with the curing agent (2) and can be reacted with the curing agent (2) and cured.

The representative examples of the base polymer (1) include, for example, hydroxyl group-containing polymers having a hydroxyl group value falling in a range of 5 to 500 mg KOH/g, preferably 20 to 300 mg KOH/g and more preferably 50 to 200 mg KOH/g and a number average molecular weight falling in a range of 500 to 50,000, preferably 1,000 to 30,000 and more preferably 1,500 to 10,000. Capable of being given as the resin kind of the base polymer (1) are, for example, acrylic resins, polyester resins, silicon acrylic resins, fluororesins and epoxy resins. Among them, particularly the acrylic resins and the polyester resins can suitably be used.

Capable of being given as the acrylic resin which can be used as the base polymer (1) are, for example, the (co)polymers of the following polymerizable unsaturated monomers having such reactive group as described above. Preferred are the polymers comprising 30 % by weight or more, preferably 40 to 90 % by weight of a $C_1$ to $C_{24}$ alkyl (meth)acrylate unit based on the whole monomer components, and the acrylic resins having a hydroxyl group are particularly suited.

The polymerizable unsaturated monomers having a reactive group include, for example, monoesters of polyhydric alcohols and acrylic acid or methacrylic acid such as 2-hydroxyethyl (meth)acrylate, hydroxypropyl (meth) acrylate, 2,3-dihydroxybutyl (meth)acrylate, 4-hydroxybutyl (meth)acrylate and polyethylene glycol mono(meth)acrylate; compounds obtained by subjecting ε-caprolactone to ring opening reaction with the monoesters described above of the polyhydric alcohols and acrylic acid or methacrylic acid; carboxyl group-containing polymerizable unsaturated monomers such as acrylic acid, methacrylic acid, itaconic acid, maleic acid and crotonic acid; acid anhydride group-containing polymerizable unsaturated monomers such as maleic anhydride and itaconic anhydride; epoxy group-containing polymerizable unsaturated monomers such as glycidyl (meth)acrylate, allyl glycidyl ether and 3,4-epoxycyclohexylmethyl (meth)acrylate; isocyanate group-containing polymerizable unsaturated monomers such as isocyanatethyl (meth)acrylate and m-propenyl-α,α-dimethylbenzylisocyanate dimethylbenzylisocyanate; and alkoxysilyl group-containing polymerizable unsaturated compounds such as vinyltrimethoxysilane, vinyltriethoxysilane, vinylmethyldimethoxysilane, vinylmethyldiethoxysilane, γ-(meth)acryloyloxypropyltrimethoxysilane, γ-(meth)acryloyloxypropylmethyldimethoxysilane, γ-(meth)acryloyloxypropylmethyldiethoxysilane, γ-(meth)acryloyloxypropyltriethoxysilane, β-(meth)acryloyloxyethyltrimethoxysilane and γ-(meth)acryloyloxybutylphenyldimethoxysilane.

The other polymerizable unsaturated monomers which can be copolymerized with these polymerizable unsaturated monomers having a reactive group include , for example, linear, branched or cyclic $C_1$ to $C_{24}$ alkyl (meth) acrylates such as methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, 2-ethylhexyl acrylate, n-octyl (meth)acrylate, lauryl (meth)acrylate, tridecyl (meth)acrylate, stearyl (meth)acrylate, isobornyl (meth)acrylate, tricyclodecanyl (meth) acrylate and cyclohexyl acrylate; nitrogen-containing( meth)acrylates such as N,N-dimethylaminoethyl (meth)acrylate, N,N-diethylaminoethyl (meth)acrylate, N,N-dimethylaminopropyl (meth)acrylate and N,N-diethylaminopropyl (meth) acrylate; acrylamide, methacrylamide; (meth)acrylamide derivatives such as N,N-dimethylaminoethyl (meth)acrylamide, N,N-diethylaminoethyl(meth)acrylamide, N,N-dimethylaminopropyl(meth)acrylamide, N,N-diethylaminopropyl (meth)acrylamide, N-methylolacrylamide, N-methoxymethylacrylamide and N-methoxybutylacrylamide; vinyl aromatic compounds such as styrene, vinyltoluene and α-methylstyrene; acrylonitrile, methacrylonitrile, vinyl acetate and Beova Monomer (manufactured by Shell Chemical Co., Ltd.).

These polymerizable unsaturated monomers can be used alone or in combination of two or more kinds thereof.

The polyester resin capable of being used as the base polymer (1) includes, for example, oil-free polyester resins, oil-modified alkyd resins; and modified products of these resins, for example, urethane-modified polyester resins, urethane-modified alkyd resins and epoxy-modified polyester resins, and the polyester resins having a hydroxyl group are suited.

The oil-free polyester resin described above comprises an ester of a polybasic acid component and a polyhydric alcohol component. Mainly used as the polybasic acid component are, for example, at least one dibasic acid selected from phthalic anhydride, isophthalic acid, terephthalic acid, tetrahydrophthalic anhydride, hexahydrophthalic anhydride, hexahydroterephthalic anhydride, succinic acid, fumaric acid, adipic acid, sebacic acid and maleic anhydride, and lower alkyl esters of these acids. Capable of being used in combination are, if necessary, monobasic acids such as benzoic acid, crotonic acid and p-t-butylbenzoic acid, and trivalent or higher polybasic acids such as trimellitic anhydride, methylcyclohexenetricarboxylic acid and pyromellitic anhydride. Further, mainly used as the polyhydric alcohol component are, for example, dihydric alcohols such as ethylene glycol, diethylene glycol, propylene glycol, 1,4-butanediol, neopentyl glycol, 3-methylpentanediol, 1,4-hexanediol, 1,6-hexanediol, 2-butyl-2-ethyl-1,3-propanediol

and 1,4-dimethylolcyclohexane. Further, capable of being used in combination are, if necessary, trihydric or higher alcohols such as glycerin, trimethylolethane, trimethylolpropane and pentaerythritol. These polyhydric alcohols can be used alone or in a mixture of two or more kinds thereof. Esterification or transesterification between these both components can be carried out by a conventionally known method. Isophthalic acid, terephthalic acid and lower alkyl esters of these acids are particularly preferred as the acid component.

The alkyd resins are obtained by reacting the acid components and the alcohol components of the oil-free polyester resins described above with oil fatty acids by a conventionally known method. Capable of being given as the oil fatty acids are, for example, coconut oil fatty acid, soybean oil fatty acid, linseed oil fatty acid, safflower oil fatty acid, tall oil fatty acid, dehydrated castor oil fatty acid and tung oil fatty acid. An oil length of the alkyd resins is preferably 30 % or less, particularly 5 to 20 %.

The urethane-modified polyester resins include resins obtained by reacting the oil-free polyester resins described above or oil-free polyester resins of a low molecular weight obtained by reacting the acid components and the alcohol components used in producing the oil-free polyester resins described above with polyisocyanate compounds by a conventionally known method. Also, the urethane-modified alkyd resin includes resins obtained by reacting the alkyd resins described above or alkyd resins of a low molecular weight obtained by reacting the respective components used in producing the alkyd resins described above with polyisocyanate compounds by a conventionally known method.

The polyisocyanate compounds which can be used in producing the modified polyester resins and the modified alkyd resins each described above include, for example, aliphatic, alicyclic, aromatic or aromatic/aliphatic polyisocyanate compounds such as hexamethylenediisocyanate, isophoronediisocyanate, xylilenediisocyanate, tolylenediisocyanate, 4,4'-diphenylmethanediisocyanate, 4,4'-methylenebis(cyclohexylisocyanate) and 2,4,6-triisocyanatotoluene.

In general, capable of being suitably used as the urethane-modified resins described above are the resins having such a modification degree that these polyisocyanate compounds constituting the urethane-modified resins account for 30 % by weight or less, particularly 1 to 20 % by weight based on the urethane-modified resins.

The epoxy-modified polyester resins include, for example, reaction products obtained by using polyester resins produced from the respective components used for producing the polyester resins described above and reacting carboxyl groups in the polyester resins with epoxy group-containing resins and reaction products obtained by bonding hydroxyl groups contained in polyester resins with hydroxyl groups contained in epoxy resins via polyisocyanate compounds by addition, condensation or graft reaction of the polyester resins with the epoxy resins. In general, the epoxy-modified polyester resins have suitably such a modification degree that the epoxy resins account for 0.1 to 30 % by weight, particularly 1 to 20 % by weight based on the epoxy-modified polyester resins.

The silicon acrylic resins which can be used as the base polymer (1) include, for example, resins obtained by subjecting hydroxyl group-containing acrylic resins to condensation reaction with silicon resins having a hydroxyl group or an alkoxyl group by dehydration or dealcohol by heating, if necessary, in the presence of a reaction catalyst such as a metal alkoxide compound.

The silicon polyester resins which can be used as the base polymer (1) include, for example, resins obtained by subjecting hydroxyl group-containing polyester resins to condensation reaction with silicon resins having a hydroxyl group or an alkoxyl group by dehydration or dealcohol by heating, if necessary, in the presence of a reaction catalyst such as a metal alkoxide compound.

The silicon resin having a hydroxyl group or an alkoxyl group used for producing the silicon acrylic resins and the silicon polyester resins each described above can be obtained in the form of commercial products, and capable of being given as the examples thereof are, for example, SH-6018, DC3074, DC3037 and SR2402 (all products described above are manufactured by Toray Dow Corning Co., Ltd.); KR9218 and X-40-9220 (all products described above are manufactured by Shin-etsu Chemical Co., Ltd.), TSR165 and XR-31B1763 (all products described above are manufactured by Toshiba Silicon Co., Ltd.).

In general, a use proportion of the silicon resins described above in the silicon acrylic resins and the silicon polyester resins falls suitably in a range of 1 to 100 parts by weight, particularly 5 to 60 parts by weight per 100 parts by weight of the resin solid matters of the acrylic resins and the polyester resins.

The fluororesins which can be used as the base polymer (1) include preferably hydroxyl group-containing fluororesins, and capable of being given are, for example, copolymers of fuloroolefins such as vinyl fluoride and hydroxyl group-containing polymerizable unsaturated monomers such as hydroxyethyl vinyl ether with other polymerizable unsaturated monomers such as ethyl vinyl ether.

The epoxy resins which can be used as the base polymer (1) include, for example, glycidyl ether type epoxy resins, glycidyl ester type epoxy resins, other glycidyl type epoxy resins; and modified epoxy resins obtained by modifying these epoxy resins with modifying agents such as alkylphenols and fatty acids. These various epoxy resins may be increased in a molecular weight with a linking agent in order to obtain a suitable molecular weight.

The glycidyl ether type epoxy resins described above are epoxy resins having a glycidyl ether group which can be obtained by reacting polyhydric alcohols or polyhydric phenols with epihalohydrins or alkylene oxides. The examples of the polyhydric alcohols described above include ethylene glycol, diethylene glycol, triethylene glycol,

polyethylene glycol, propylene glycol, polypropylene glycol, neopentyl glycol, butylene glycol, 1,6-hexanediol, 1,4-hexanediol, glycerin, trimethylolethane, trimethylolpropane, pentaerythritol, diglycerin and sorbitol. The examples of the polyhydric phenols described above-include 2,2-bis(4-hydroxyphenyl)-propane (bisphenol A), 2,2-bis(2-hydroxyphenyl)propane, 2-(2-hydroxyphenyl)-2-(4-hydroxyphenyl)propane, halogenated bisphenol A, bis(4-hydroxyphenyl)methane (bisphenol F), 4,4'-dihydroxybenzophenone, tris(4-hydroxyphenyl)propane, 1,1-bis(4-hydroxyphenyl)ethane, 4,4'-dihydroxybiphenol, resorcin, hydroquinone, tetrahydroxyphenylethane, 1,2,3-tris(2,3-epoxypropoxy)propane, novolak type polyhydric phenols and cresol type polyhydric phenols.

The glycidyl ester type epoxy resins described above include resins obtained by highly polymerizing diglycidyl phthalate, diglycidyl hexahydrophthalate, diglycidyl tetrahydrophthalate and dimeric acid diglycidyl ester by using linking agents.

The other glycidyl type epoxy resins described above include, for example, resins obtained by highly polymerizing tetraglycidylaminodiphenylmethane and triglycidylisocyanurate by using linking agents.

The modified epoxy resins are epoxy resins obtained by modifying epoxy resins before modified such as the glycidyl ether type epoxy resins and the glycidyl ester type epoxy resins each described above with modifying agents such as alkylphenols and fatty acids.

The linking agents used in order to optimize the molecular weights of the various epoxy resins such as the glycidyl ether type epoxy resins, the glycidyl ester type epoxy resins and the other glycidyl type epoxy resins each described above include, for example, polyhydric phenols, polyhydric alcohols, polybasic acids, primary or secondary amines and polyisocyanates. The polyhydric alcohols and polyhydric phenols which have been given above as the examples of the raw materials for the glycidyl ether type epoxy resins can be given as the polyhydric phenols and polyhydric alcohols. The polybasic acids include, for example, adipic acid, azelaic acid, sebacic acid, hexahydrophthalic anhydride, tetrahydrophthalic anhydride, isophthalic acid and dimeric acid. The primary or secondary amines include, for example, ethylamine, n-propylamine, isopropylamine, n-butylamine, monoethanolamine and hexamethylenediamine. The polyisocyanates include, for example, isophoronediisocyanate, diphenylmethanediisocyanate and hexamethylenediisocyanate.

Curing agent (2):

The curing agent which is the component (2) in the curable composition II of the present invention is a substance which has a reactivity with a hydroxyl group and which can be reacted with a hydroxyl group produced by hydrolysis of an orthoester group in the polyorthoester (3) described above and also reacted with the base polymer (1) to form a cured matter.

Capable of being given as the representative example of the curing agent (2) are, for example, polyisocyanate compounds, amino resins, epoxy group-containing compounds, alkoxysilyl group-containing compounds and compounds having two or more carboxylic anhydride groups.

The same ones as the examples of the compound (B) explained above as the curing agent (B) in the curable composition I of the present invention can be given as the specific examples of these polyisocyanate compounds, amino resins, epoxy group-containing compounds, alkoxysilyl group-containing compounds and compounds having two or more carboxylic anhydride groups.

Curable composition:

The curable composition II of the present invention comprises a combination of the base polymer (1) and the curing agent (2) each described above and further comprises the polyorthoester of the present invention described above. A blending proportion of the respective components (1), (2) and (3) described above shall not specifically be restricted, and usually, it falls suitably in the following range based on 100 parts by weight of the total of solid matters contained in the components (1), (2) and (3):

component (1): 20 to 89 parts by weight, preferably 25 to 87 parts by weight and more preferably 30 to 85 parts by weight,
component (2): 5 to 70 parts by weight, preferably 10 to 60 parts by weight and more preferably 15 to 55 parts by weight, and
component (3): 1 to 40 parts by weight, preferably 3 to 25 parts by weight and more preferably 5 to 25 parts by weight.

The curable composition II of the present invention can suitably contain, if necessary, an acid catalyst, an organic solvent, a curing catalyst, a pigment, a UV absorber, a coated surface-controlling agent, an antioxidant, a fluidity-controlling agent and a wax in addition to the components (1), (2) and (3) described above.

The acid catalyst described above is a catalyst for accelerating a reaction of deblocking an orthoester group to reproduce a hydroxyl group. The kind thereof shall not specifically be restricted, and capable of being used as well are, for example, those which have been given as the examples of the acid catalyst capable of being blended with the curable composition I of the present invention.

The curable composition II of the present invention can usually be a composition of a non-solvent or organic solvent type. When it is the composition of an organic solvent type, solvents which can dissolve or disperse the respective components of the curable composition II can be used and include, for example, the same ones as given as the examples of the organic solvents for the curable composition I.

Further, suitable ones selected from those given above as the examples of the curing catalyst and the pigment which can be blended with the curable composition I of the present invention can also be used as the curing catalyst and the pigment which are blended with the curable composition II of the present invention.

The curing conditions of the curable composition II of the present invention can be changed according to the kind of the curing agent (2) used and shall not specifically be restricted. Usually, when the curing time is 5 minutes or longer, the curing temperature falls suitably in a range of a room temperature (about 0°C) to about 200°C, particularly about 60°C to about 180°C, and when the curing time is shorter than 5 minutes, the curing temperature falls suitably in a range of about 60°C to about 300°C, particularly about 80°C to about 260°C.

The curable composition I of the present invention can suitably be used for uses in coating material compositions, adhesives and inks, particularly as a coating material composition.

## Examples

The present invention shall more specifically be explained below with reference to examples and comparative examples, and "parts" and "%" mean "parts by weight" and "% by weight" respectively.

<u>Production of polyester solution</u>

Production Example 1

A reactor equipped with a stirrer, a condenser, a temperature-controlling device, a water separator, a fractionating column, a nitrogen-introducing tube and a solvent-recovering device was charged with 161 parts of 1,6-hexanediol, 351 parts of 1,4-dimethylolcyclohexane, 146 parts of trimethylolpropane, 114 parts of adipic acid, 300 parts of hexahydrophthalic anhydride and 243 parts of isophthalic acid, and the reactor was substituted with nitrogen and then started to be heated. The temperature was elevated at a fixed rate from 170°C to 230°C in 3 hours while removing condensation water and then maintained at 230°C for one hour. Thereafter, 50 parts of xylene was added, and the reaction was further promoted for 3 hours while maintaining at 230°C and removing condensation water by means of the water separator. Then, the reactor was cooled down, and 464 parts of xylene was added thereto to obtain a polyester solution (PE-1) having a non-volatile matter content of about 69 % and a Gardner viscosity (20°C) of X. The resulting resin (solid matter) had a resin acid value of 6.5 mg KOH/g, a hydroxyl group value of 120 mg KOH/g, a number average molecular weight of 1,800 and a weight average molecular weight of 5,200.

<u>Production of acrylic resin solution</u>

Production Example 2

A reactor equipped with a stirrer, a condenser, a temperature-controlling device, a nitrogen-introducing tube and a dropping funnel was charged with 983 parts of xylene and 240 parts of 3-methoxybutyl acetate, and the reactor was substituted with nitrogen, heated and maintained at 135°C. Dropwise added thereto in 4 hours was a mixture comprising 600 parts of styrene, 636 parts of isobutyl methacrylate, 552 parts of 2-ethylhexyl acrylate, 612 parts of 2-hydroxyethyl methacrylate and 192 parts of azobisisobutyronitrile. After finishing dropwise adding, the solution was ripened at 135°C for 30 minutes, and then a mixed solution comprising 168 parts of xylene and 12 parts of azobisisobutyronitrile was dropwise added thereto in one hour. Then, the solution was maintained at 135°C for 30 minutes to obtain an acrylic resin solution (AR-1) having a non-volatile matter content of about 63 % and a Gardner viscosity (20°C) of $U^+$. The resulting resin (solid matter) had a hydroxyl value of 110 mg KOH/g, a number average molecular weight of 1,900 and a weight average molecular weight of 4,300.

Production of polyorthoester

Example 1

A reactor equipped with a stirrer, a condenser, a temperature-controlling device and a solvent-recovering device was charged with 424 parts of methyl orthoformate, 640 parts of 2-butyl-2-ethyl-1,3-propanediol, 136 parts of pentaerythritol and 4 parts of a 90 % formic acid aqueous solution and maintained at about 85°C for one hour while distilling off methanol produced by alcohol exchange reaction. Then, the temperature was elevated up to 190°C in 2 hours, and 365 parts of methanol was recovered to obtain a colorless and liquid polyorthoester. The polyorthoester thus obtained had a Gardner viscosity of $X^+$ and a weight average molecular weight of 1,540. Examples 2 to 12 and Comparative Examples 1 to 3.

The reaction was carried out in the same manner as in Example 1 to obtain the respective polyorthoesters, except that in Example 1, the blended raw material compositions were changed as shown in the following Table 1. In Examples 6 and 7, distilled off was a part of an organic solvent (xylene) contained in the raw material in addition to alcohol produced by alcohol exchange. The polyorthoester solution obtained in Example 6 had a solid content of about 74 %, and the polyorthoester solution obtained in Example 7 had a solid content of about 68 %.

The polyorthoester obtained in Comparative Example 1 had a solid content of about 100 % and crystallized. In Comparative Example 2 and Comparative Example 3, gelation took place in the middle of the reaction.

All the polyorthoesters obtained in Examples 1 to 5 and 8 to 12 were colorless and liquid polyorthoesters and had a solid content of substantially about 100 %. In Example 6 and Comparative Example 2, the acid catalyst was not blended, but a carboxyl group contained in the resin in the polyester solution (PE-1) works as a catalyst.

A remark in Table 1 means the following:

(remark 1) Placcel 305: polycaprolactonepolyol, manufactured by Daicel Chemical Industries, Ltd.

Table 1

| | | Example | | | | | | | | | | | | Comparative Example | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 1 | 2 | 3 |
| Component (A) | Methyl orthoformate | 424 | | | | | 106 | | | 424 | 636 | 424 | 424 | 212 | 35 | |
| | Methyl orthoacetate | | | 480 | 480 | 480 | | 120 | 360 | | | | | | | 40 |
| | Ethyl orthoformate | | 592 | | | | | | | | | | | | | |
| Component (B) | 2-Butyl-2-ethyl-1,3-propanediol | 640 | 640 | 640 | | | 160 | 160 | | 320 | 160 | 640 | | | | |
| | Neopentyl glycol | | | | 416 | | | | 312 | | | | | | | |
| | 2,2,4-trimethyl-1,3-pentanediol | | | | | 584 | | | | | | | | | | |
| | 1,2-Hexanediol | | | | | | | | | | | | 472 | | | |

To be continued

Table 1 (continued)

| | | Example | | | | | | | | | | | | Comparative Example | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 1 | 2 | 3 |
| Component (C) | Pentaerythritol | 136 | 136 | 136 | 136 | 136 | | | | 68 | 136 | 136 | 136 | 136 | | |
| | Polyester solution (PE-1) | | | | | | 668 | | | | | | | | 668 | |
| | Acrylic resin solution (AR-1) | | | | | | | 850 | | | | | | | | 850 |
| | Placcel 305 (remark 1) | | | | | | | | 550 | | | | | | | |
| | Trimethylol-propane | | | | | | | | | 268 | 536 | | | | | |
| Acid catalyst | 90 % formic acid aqueous solution | 4 | 4 | 4 | 4 | 4 | | 2 | 3 | 8 | 6 | | 4 | 4 | | 2 |
| | p-Toluenesulfonic acid | | | | | | | | | | | 1 | | | | |
| Recovered alcohol | Methanol | 365 | | 360 | 366 | 342 | 91 | 92 | 267 | 362 | 523 | 341 | 360 | 130 | 16 | 22 |
| | Ethanol | | 539 | | | | | | | | | | | | | |
| Properties of polyorthoester (solution) | Gardner viscosity | X⁺ | W | M | G⁺ | A1⁺ | Z | UV | S | OP | W | Y | T | Crystal-lized | Gelled | Gelled |
| | Weight average molecular weight | 1540 | 1420 | 1060 | 430 | 410 | 6950 | 5620 | 1640 | 1250 | 1600 | 1590 | 1270 | — | — | — |

A weight average molecular weight, a hydroxyl group value and a viscosity of the polyorthoesters obtained in Examples 1 and 11 were compared with those of Placcel 303, and the results thereof are shown in the following Table 2.

A remark in Table 2 means the following:

(remark 2) Placcel 303: polycaprolactonepolyol, manufactured by Daicel Chemical Industries, Ltd.

Table 2

| | Weight average molecular weight | Hydroxyl group value* (mg KOH/g) | Viscosity (mPa·s) |
|---|---|---|---|
| Polyorthoester of Example 1 | 1540 | 550 | 1350 |
| Polyorthoester of Example 11 | 1590 | 550 | 1420 |
| Praccel 303 (remark 2) | 610 | 540 | 1800 |

* The hydroxyl group value represents a hydroxyl group value obtained after hydrolysis (a hydroxyl group value after recovering a hydroxyl group) in polyorthoesters.

It is apparent from the results shown in Table 1 that all the polyorthoesters obtained in Examples 2 to 5 and Examples 8 to 10 and 12 had a lower Gardner viscosity than that of the polyorthoester obtained in Example 1.

The polyorthoester obtained in Example 6 had a solid content of about 74 %, and when the solid content was controlled to 69 % by adding xylene to the above polyorthoester, the Gardner viscosity was V, which was lower than the viscosity X of the polyester solution (PE-1) having a solid content of 69 % used in Example 6.

Further, the polyorthoester solution obtained in Example 7 had a solid content of about 68 %, and when the solid content was controlled to 63 % by adding xylene to the above polyorthoester, the Gardner viscosity was O, which was lower than the viscosity $U^+$ of the acrylic resin solution (AR-1) having a solid content of 63 % used as a raw material in Example 7.

<u>Production of compound having two or more groups having reactivity with a hydroxyl group in a molecule</u>

Production Example 3

A reactor equipped with a stirrer, a condenser, a temperature-controlling device, a nitrogen-introducing tube and a dropping funnel was charged with 983 parts of xylene and 240 parts of 3-methoxybutyl acetate, and the reactor was substituted with nitrogen, heated and maintained at 135°C. Dropwise added thereto in 4 hours was a mixture comprising 720 parts of styrene, 720 parts of 2-ethylhexyl acrylate, 960 parts of glycidyl methacrylate and 192 parts of azobisisobutyronitrile. After finishing dropwise adding, the solution was aged at 135°C for 30 minutes, and then a mixed solution comprising 168 parts of xylene and 12 parts of azobisisobutyronitrile was dropwise added in one hour. Then, the solution was maintained at 135°C for 30 minutes to obtain an epoxy group-containing acrylic resin solution (B-1) having a non-volatile matter content of about 63 % and a Gardner viscosity (20°C) of S. The resulting resin (solid matter) had an epoxy equivalent of about 370, a number average molecular weight of 2,100 and a weight average molecular weight of 4,900.

Production Example 4

A reactor equipped with a stirrer, a condenser, a temperature-controlling device, a nitrogen-introducing tube and a dropping funnel was charged with 983 parts of xylene and 240 parts of 3-methoxybutyl acetate, and the reactor was substituted with nitrogen, heated and maintained at 135°C. Dropwise added thereto in 4 hours was a mixture comprising 600 parts of styrene, 600 parts of maleic anhydride, 1200 parts of n-butyl acrylate and 192 parts of azobisisobutyronitrile. After finishing dropwise adding, the solution was aged at 135°C for 30 minutes, and then a mixed solution comprising 168 parts of xylene and 12 parts of azobisisobutyronitrile was dropwise added in one hour. Then, the solution was maintained at 135°C for 30 minutes to obtain an acid anhydride group-containing acrylic resin solution (B-2) having a non-volatile matter content of about 63 % and a Gardner viscosity (20°C) of $R^-$. The resulting resin (solid matter) had a whole acid value of about 266 mg KOH/g, a half acid value of about 138 mg KOH/g, a number average molecular weight of 1,900 and a weight average molecular weight of 4,800.

Production Example 5

A reactor equipped with a stirrer, a condenser, a temperature-controlling device, a nitrogen-introducing tube and a dropping funnel was charged with 983 parts of xylene and 240 parts of 3-methoxybutyl acetate, and the reactor was substituted with nitrogen, heated and maintained at 135°C. Dropwise added thereto in 4 hours was a mixture comprising 720 parts of styrene, 720 parts of n-butyl methacrylate, 480 parts of 2-ethylhexyl acrylate, 480 parts of γ-methacryloyloxypropyltrimethoxysilane and 192 parts of azobisisobutyronitrile. After finishing dropwise adding, the solution was aged at 135°C for 30 minutes, and then a mixed solution comprising 168 parts of xylene and 12 parts of azobisisobutyronitrile was dropwise added in one hour. Then, the solution was maintained at 135°C for 30 minutes to obtain an alkoxysilyl group-containing acrylic resin solution (B-3) having a non-volatile matter content of about 63 % and a Gardner viscosity (20°C) of W. The resulting resin (solid matter) had a number average molecular weight of 2,800 and a weight average molecular weight of 5,500.

Production Example 6

A reactor equipped with a stirrer, a condenser, a temperature-controlling device, a nitrogen-introducing tube and a dropping funnel was charged with 983 parts of xylene and 240 parts of 3-methoxybutyl acetate, and the reactor was substituted with nitrogen, heated and maintained at 135°C. Dropwise added thereto in 4 hours was a mixture comprising 480 parts of styrene, 480 parts of maleic anhydride, 480 parts of glycidyl methacrylate, 960 parts of 2-ethylhexyl acrylate and 192 parts of azobisisobutyronitrile. After finishing dropwise adding, the solution was aged at 135°C for 30 minutes, and then a mixed solution comprising 168 parts of xylene and 12 parts of azobisisobutyronitrile was dropwise added in one hour. Then, the solution was maintained at 135°C for 30 minutes to obtain an acrylic resin solution (B-4) containing an epoxy group and acid anhydride group and having a non-volatile matter content of about 63 % and a Gardner viscosity (20°C) of $S^+$. The resulting resin (solid matter) had an epoxy equivalent of about 710, a half acid value of about 102 mg KOH/g, a number average molecular weight of 2,200 and a weight average molecular weight of 4,900.

<u>Preparation of curable composition I</u>

Example 13

The polyorthoester 100 parts obtained in Example 1 was blended with 191 parts of Desmodur N-3300 (triisocyanurate of hexamethylenediisocyanate, manufactured by Sumitomo Bayer Urethane Co., Ltd.) and 11.6 parts of Nacure 5543 (sulfonate base acid catalyst solution, active ingredient: about 25 %, manufactured by King Industries Inc., U.S.A.), and the solution was homogeneously mixed to obtain a curable composition.

Examples 14 to 34 and Comparative Examples 4 to 5

The same operation as in Example 13 was carried out to obtain the respective curable compositions, except that in Example 13, the blending composition was changed as shown in the following Table 3.

The remarks in Table 3 mean the following respectively: (remark 1) and (remark 2) mean the same as those described above (remark 3) Cymel 303: methyl-etherified melamine resin, solid content: about 100 %, manufactured by Mitsui Cytec Co., Ltd.) (remark 4) Cymel 325: methyl-etherified melamine resin, solid content: about 80 %, manufactured by Mitsui Cytec Co., Ltd.) (remark 5) SB-20AH: polyacid anhydride of ethyl-octadecanedioic acid, condensation degree: about 4.1, manufactured by Okamura Oil Mill, Ltd.).

The respective curable compositions obtained in Examples 13 to 34 and Comparative Examples 4 to 5 were tested for a compatibility and a solvent resistance and a gel ratio of the cured coating films based on the following test methods. Further, the respective curable compositions obtained in Examples 13 to 34 and Comparative Examples 4 to 5 were tested as well for a pot life. The test results thereof are shown in the following Table 3. Further, the Gardener viscosities of the respective curable compositions obtained in Examples 13 to 34 and Comparative Examples 4 to 5 are shown as well in Table 3.

<u>Test methods</u>

Compatibility:

Each curable composition was put in a test tube to visually observe the appearance thereof, and it was evaluated according to the following criteria:

O: not cloudy and transparent, and compatibility is good
Δ: cloudy, and compatibility is inferior
×: phases are separated, and compatibility is notably inferior

Solvent resistance:

The curable composition was coated on a polished cold rolled steel plate so that a dried film thickness was about 30μm and dried at 140°C for 30 minutes (only the composition prepared in Example 30 was dried at 160°C for 30 minutes) to obtain a coating film. The surface of the coated film was rubbed in a length of about 5 cm at a load of about 1 kg/cm$^2$ by 20 reciprocations with a three-folded gauze impregnated with xylene, and then the state of the surface of the coated film was observed and evaluated according to the following criteria:

O: coating film has no scratches and dull glossiness and is of a good state
Δ: coating film has slightly scratches or slightly dull glossiness and is of a little inferior state
×: coating film is dissolved or markedly scratched

Gel ratio:

The curable composition was coated on a teflon sheet so that a dried film thickness was about 30μm and dried at 140°C for 30 minutes (only the composition prepared in Example 30 was dried at 160°C for 30 minutes), and the coating film was peeled off to obtain a free coating film. The free coating film was extracted under refluxing in acetone for 6 hours, and the gel ratio (%) was determined from the coating film weights before and after extraction according to the following equation:

$$\text{gel ratio} = \frac{\text{(coating film weight after extraction)}}{\text{(coating film weight before extraction)}} \times 100$$

Pot life:

The curable composition was put in a glass bottle of 100 ml, and the bottle was sealed up and left standing in a dark place of 30°C to measure the viscosity every 6 hours to determine time passing until the viscosity grew larger to lose fluidity:

O: fluidity is kept even after passage of 24 hours
Δ: fluidity is kept after 6 hours, but fluidity is lost after 24 hours
×: fluidity is lost within 6 hours

Table 3

| | | Example | | | | | | | | | | | | Comparative Example | |
|---|---|---|---|---|---|---|---|---|---|---|---|---|---|---|---|
| | | 13 | 14 | 15 | 16 | 17 | 18 | 19 | 20 | 21 | 22 | 23 | 24 | 4 | 5 |
| Polyortho-ester | Kind (Production Example No.) | 1 | 2 | 3 | 4 | 5 | 6 | 7 | 8 | 9 | 10 | 11 | 12 | | |
| | Amount | 100 | 100 | 100 | 100 | 100 | 131 | 143 | 100 | 100 | 100 | 100 | 100 | | |
| Praccel 305 (remark 1) | | | | | | | | | | | | | | | 100 |
| Praccel 303 (remark 2) | | | | | | | | | | | | | | 100 | |
| Desmodur N3300 | | 191 | | 181 | 240 | 191 | 62 | 57 | 127 | 227 | 265 | 191 | | 187 | 106 |
| Lysinetriisocyanate | | | 90 | | | | | | | | | | 105 | | |
| Cymel 303 (remark 3) | | | | | | | | | | | | | | | |
| Cymel 325 (remark 4) | | | | | | | | | | | | | | | |
| SB-20AH (remark 5) | | | | | | | | | | | | | | | |

To be continued

Table 3 (continued)

| | | Example | | | | | | | | | | | | Comparative Example | |
|---|---|---|---|---|---|---|---|---|---|---|---|---|---|---|---|
| | | 13 | 14 | 15 | 16 | 17 | 18 | 19 | 20 | 21 | 22 | 23 | 24 | 4 | 5 |
| Acrylic resin solution having a non-volatile matter content of 63 % | (B-1) | | | | | | | | | | | | | | |
| | (B-2) | | | | | | | | | | | | | | |
| | (B-3) | | | | | | | | | | | | | | |
| | (B-4) | | | | | | | | | | | | | | |
| Nacure 5543 | | 11.6 | 7.6 | 11.2 | 13.6 | 11.6 | 6.5 | 6.3 | 9.1 | 13.1 | 14.6 | 11.6 | 8.2 | 11.5 | 8.2 |
| Lead 2-ethylhexanoate | | | | | | | | | | | | | | | |
| Tetrabutylammonium bromide | | | | | | | | | | | | | | | |
| Test results | Gardner viscosity (25°C) | V | F | Q | KL | G⁻ | Y | T⁺ | S | QR | UV | V⁺ | C⁻ | Z | W |
| | Compatibility | O | O | O | O | O | O | O | O | O | O | O | O | × | Δ |
| | Solvent resistance | O | O | O | O | O | O | O | O | O | O | O | O | × | Δ |
| | Gel ratio (%) | 97 | 99 | 96 | 98 | 95 | 95 | 97 | 99 | 99 | 98 | 99 | 98 | 85 | 96 |
| | Pot life | O | O | O | O | O | O | O | O | O | O | O | O | × | × |

To be continued

Table 3 (continued)

| | | Example | | | | | | | | | |
|---|---|---|---|---|---|---|---|---|---|---|---|
| | | 25 | 26 | 27 | 28 | 29 | 30 | 31 | 32 | 33 | 34 |
| Polyorthoester | Kind (Production Example No.) | 1 | 3 | 8 | 4 | 1 | 1 | 1 | 7 | 1 | 1 |
| | Amount | 100 | 100 | 100 | 100 | 100 | 20 | 20 | 143 | 20 | 20 |
| Placcel 305 (remark 1) | | | | | | | | | | | |
| Placcel 303 (remark 2) | | | | | | | | | | | |
| Desmodur N3300 | | | | | | 191 | | | | | |
| Lysinetriisocyanate | | | | | | | | | | | |
| Cymel 303 (remark 3) | | 67 | 67 | 67 | | 20 | | | | | |
| Cymel 325 (remark 4) | | | | | 84 | | | | | | |
| SB-20AH (remark 5) | | | | | | | | | 20 | | |

To be continued

Table 3 (continued)

| | | Example 25 | 26 | 27 | 28 | 29 | 30 | 31 | 32 | 33 | 34 |
|---|---|---|---|---|---|---|---|---|---|---|---|
| Acrylic resin solution having a non-volatile matter content of 63 % | (B-1) | | | | | | 123 | | | | |
| | (B-2) | | | | | | | 123 | | | |
| | (B-3) | | | | | | | | | 123 | |
| | (B-4) | | | | | | | | | | 123 |
| Nacure 5543 | | 6.7 | 6.7 | 6.7 | 6.7 | 12.4 | 4 | 4 | 4.8 | 4 | 4 |
| Lead 2-ethylhexanoate | | | | | | | 3 | | | | |
| Tetrabutylammonium bromide | | | | | | | | 1.5 | 1.5 | | 1.5 |
| Test Result | Compatibility | O | O | O | O | O | O | O | O | O | O |
| | Solvent resistance | O | O | O | O | O | O | O | O | O | O |
| | Gel ratio (%) | 99 | 99 | 99 | 99 | 99 | 94 | 95 | 97 | 98 | 99 |

Production of acrylic resin solution

Production Example 7

A reactor equipped with a stirrer, a condenser, a temperature-controlling device, a nitrogen-introducing tube and a dropping funnel was charged with 983 parts of xylene and 240 parts of 3-methoxybutyl acetate, and the reactor

was substituted with nitrogen, heated and maintained at 135°C. Dropwise added thereto in 4 hours was a mixture comprising 600 parts of styrene, 384 parts of isobutyl methacrylate, 480 parts of 2-ethylhexyl acrylate, 552 parts of 2-hydroxyethyl methacrylate, 450 parts of Placcel FM-3X (80 % xylene solution, manufactured by Daicel Chemical Industries, Ltd.), 24 parts of acrylic acid and 120 parts of azobisisobutyronitrile. After finishing dropwise adding, the solution was aged at 135°C for 30 minutes, and then a mixed solution comprising 144 parts of xylene and 24 parts of azobisisobutyronitrile was dropwise added in one hour. Then, the solution was maintained at 135°C for 30 minutes to obtain an acrylic resin solution (AR-2) having a non-volatile matter content of about 60 % and a Gardner viscosity (20°C) of T. The resulting resin (solid matter) had a hydroxyl value of about 117 mg KOH/g, a number average molecular weight of 3,400 and a weight average molecular weight of 7,500.

Production Example 8

A reactor equipped with a stirrer, a condenser, a temperature-controlling device, a nitrogen-introducing tube and a dropping funnel was charged with 983 parts of xylene and 240 parts of 3-methoxybutyl acetate, and the reactor was substituted with nitrogen, heated and maintained at 135°C. Dropwise added thereto in 4 hours was a mixture comprising 240 parts of styrene, 240 parts of methyl methacrylate, 720 parts of n-butyl methacrylate, 240 parts of 2-hydroxyethyl methacrylate, 960 parts of glycidyl methacrylate and 120 parts of azobisisobutyronitrile. After finishing dropwise adding, the solution was aged at 135°C for 30 minutes, and then a mixed solution comprising 144 parts of xylene and 24 parts of azobisisobutyronitrile was dropwise added in one hour. Then, the solution was maintained at 135°C for 30 minutes and diluted with 234 parts of xylene to obtain an acrylic resin solution (AR-3) having a non-volatile matter content of about 60 % and a Gardner viscosity (20°C) of RS. The resulting resin (solid matter) had a hydroxyl value of about 43 mg KOH/g, an epoxy equivalent of about 360, a number average molecular weight of about 3,000 and a weight average molecular weight of 6,900.

Production Example 9

A reactor equipped with a stirrer, a condenser, a temperature-controlling device, a nitrogen-introducing tube and a dropping funnel was charged with 288 parts of xylene and 432 parts of 3-methoxybutyl acetate, and the reactor was substituted with nitrogen, heated and maintained at 135°C. Dropwise added thereto in 4 hours was a mixture comprising 701 parts of styrene, 463 parts of isobutyl methacrylate, 636 parts of isobutyl acrylate, 600 parts of maleic anhydride and 720 parts of 3-methoxybutyl acetate, and a mixture comprising 264 parts of t-butylperoxy-2-ethylhexanoate and 672 parts of xylene was dropwise added at the same time therewith in 4 hours and 30 minutes to obtain an acrylic resin solution (AR-4) having a non-volatile matter content of about 56 % and a Gardner viscosity (20°C) of ST. The resulting resin (solid matter) had a whole acid value of about 270, a number average molecular weight of 1,900 and a weight average molecular weight of 4,100.

<u>Production of polyester solution</u>

Production Example 10

A reactor equipped with a stirrer, a condenser, a temperature-controlling device, a water separator, a fractionating column, a nitrogen-introducing tube and a solvent-recovering device was charged with 200 parts of neopentyl glycol, 101 parts of trimethylolpropane, 132 parts of isophthalic acid, 116 parts of adipic acid, 61 parts of hexahydrophthalic anhydride and 68 parts of 1,4-cyclohexanedicarboxylic acid, and the reactor was substituted with nitrogen and then started to be heated. The temperature was elevated at a fixed rate from 170°C to 230°C in 3 hours while removing condensation water and then maintained at 230°C for one hour. Thereafter, 30 parts of xylene was added thereto, and the reaction was further promoted for 7 hours while maintaining at 230°C and removing condensation water by means of the water separator. Then, the reactor was cooled down, and 370 parts of xylene was added thereto to obtain a polyester solution (PE-2) having a non-volatile matter content of about 60 % and a Gardner viscosity (20°C) of V. The resulting resin (solid matter) had a resin acid value of about 10 mg KOH/g, a hydroxyl value of about 128 mg KOH/g, a number average molecular weight of 2,300 and a weight average molecular weight of 12,000.

<u>Production of polyorthoester</u>

Example 35

A reactor equipped with a stirrer, a condenser, a temperature-controlling device and a solvent-recovering device was charged with 636 parts of methyl orthoformate, 960 parts of 2-butyl-2-ethyl-1,3-propanediol, 254 parts of

dipentaerythritol and 2 parts of a 90 % formic acid aqueous solution and maintained at about 85°C for one hour while distilling off methanol produced by alcohol exchange reaction. Then, the temperature was elevated up to 190°C in 2 hours, and 553 parts of methanol was recovered to obtain a colorless and liquid polyorthoester (C-5). The polyorthoester (C-5) thus obtained had a Gardner viscosity of $Z_4$ and a weight average molecular weight of 2,010.

The polyorthoesters thus obtained was a colorless and liquid polyorthoester and had a solid content of substantially about 100 %.

Preparation of curable composition II

Example 36

The 60 % acrylic resin solution (AR-2) 16.7 parts obtained in Example 7 was blended with 9.9 parts of Desmodur N-3300 (triisocyanurate of hexamethylenediisocyanate, manufactured by Sumitomo Bayer Urethane Co., Ltd.), 3.0 parts of the polyorthoester obtained in Example 1, 4.0 parts of xylene, 0.7 part of 3-methoxybutyl acetate and 0.9 part of Nacure 5543 (sulfonic acid base acid catalyst solution, active ingredient: about 25 %, manufactured by King Industries Inc., U.S.A.), and the solution was homogeneously mixed to obtain a curable composition having a non-volatile matter content of 65 %.

Examples 37 to 46 and Comparative Examples 6 to 10

The same operation as in Example 36 was carried out to obtain the respective curable compositions, except that in Example 36, the blending composition was changed as shown in the following Table 4. The curable compositions obtained in Example 46 and Comparative Example 10 had a non-volatile matter content of 60 %, and all the curable compositions obtained in Examples 37 to 45 and Comparative Examples 6 to 9 had a non-volatile matter content of 65 %.

The remark in Table 4 means the following:

(remark 6) Cymel 303: methyl-etherified melamine resin, solid content: about 100 %, manufactured by Mitsui Cytec Co., Ltd.

The respective curable compositions obtained in Examples 36 to 46 and Comparative Examples 6 to 10 were tested for a gel ratio, a coating film hardness and a solvent resistance of the cured coating films based on the following test methods. The test results thereof are shown in the following Table 4. Further, the Gardener viscosities of the respective curable compositions obtained in Examples 36 to 46 and Comparative Examples 6 to 10 are shown as well in Table 4. All the respective curable compositions were excellent in a compatibility and a wetting property to the base material.

Test methods

Gel ratio:

The curable composition was coated on a teflon sheet so that a dried film thickness was about 30µm and dried at 140°C for 30 minutes, and the coating film was peeled off to obtain a free coating film. The free coating film was extracted under refluxing in acetone for 6 hours, and the gel ratio (%) was determined from the coating film weights before and after extraction according to the following equation:

$$\text{gel ratio} = \frac{\text{(coating film weight after extraction)}}{\text{(coating film weight before extraction)}} \times 100$$

Coating film hardness:

The curable composition was coated on a polished cold rolled steel plate so that a dried film thickness was about 30µm and dried at 140°C for 30 minutes to obtain a coating film. The coating film thus obtained was measured for a Knoop hardness at 20°C on the conditions of a load of 25 g and a load-applying time of 20 seconds by means of a microhardness meter ("Model DMH-2", a product manufactured by Matsuzawa Seiki Co., Ltd.). The larger the value, the higher the hardness.

Solvent resistance:

The curable composition was coated on a polished cold rolled steel plate so that a dried film thickness was about 30μm and dried at 140°C for 30 minutes to obtain a coating film. The surface thereof was rubbed in a length of about 5 cm at a load of about 1 kg/$cm^2$ by 20 reciprocations with a three-folded gauze impregnated with xylene, and then the state of the coated face was observed and evaluated according to the following criteria:

O: coating film has no scratches and dull glossiness and is of a good state
Δ: coating film has slightly scratches or slightly dull glossiness and is of a little inferior state
×: coating film is dissolved or markedly scratched

Table 4

| | | Example | | | | | | | | | | | Comparative Example | | | | |
|---|---|---|---|---|---|---|---|---|---|---|---|---|---|---|---|---|---|
| | | 36 | 37 | 38 | 39 | 40 | 41 | 42 | 43 | 44 | 45 | 46 | 6 | 7 | 8 | 9 | 10 |
| Base polymer | Acrylic resin solution AR-2 | 16.7 | 16.7 | 16.7 | 16.7 | 16.7 | 16.7 | 16.7 | | | | | 16.7 | 16.7 | | | |
| | Acrylic resin solution AR-3 | | | | | | | | | | | 16.7 | | | | | 16.7 |
| | Polyester solution PE-2 | | | | | | | | 16.7 | 16.7 | 16.7 | | | | 16.7 | 16.7 | |
| Curing agent | Desmodur N-3300 | 9.9 | 9.5 | 8.7 | 9.2 | 9.7 | | | 10.3 | | | | 4.1 | | 4.5 | | |
| | Cymel 303 (remark 6) | | | | | | 4 | 4 | | 4 | 4 | | | 4 | | 4 | |
| | Acrylic resin solution AR-4 | | | | | | | | | | | 16.7 | | | | | 16.7 |
| Polyorthoeste | Example 1 | 3 | | | | | 3 | | 3 | 3 | | 3 | | | | | |
| | Example 3 | | 3 | | | | | 3 | | | | | | | | | |
| | Example 4 | | | 2 | | | | | | | 3 | | | | | | |
| | Example 8 | | | | 4 | | | | | | | | | | | | |
| | Example 35 | | | | | 3 | | | | | | | | | | | |

To be continued

Table 4 (continued)

| | | Example | | | | | | | | | | | Comparative Example | | | | |
|---|---|---|---|---|---|---|---|---|---|---|---|---|---|---|---|---|---|
| | | 36 | 37 | 38 | 39 | 40 | 41 | 42 | 43 | 44 | 45 | 46 | 6 | 7 | 8 | 9 | 10 |
| Solvents | Xylene | 4.0 | 3.8 | 3.1 | 4.2 | 3.9 | 1.5 | 1.5 | 1.9 | 1.8 | 1.8 | 1.0 | 0.2 | 0.2 | 0.5 | 0.2 | |
| | 3-Methoxybutyl acetate | 0.7 | 0.7 | 0.5 | 0.7 | 0.7 | 0.3 | 0.3 | | | | | | | | | |
| Solvents | Nacure 5543 | 0.9 | 0.9 | 0.8 | 0.9 | 0.9 | 0.7 | 0.7 | 0.9 | 0.7 | 0.7 | 0.9 | 0.6 | 0.6 | 0.6 | 0.6 | 0.8 |
| | Dodecyltributylammonium bromide | | | | | | | | | | | 0.2 | | | | | 0.2 |
| Test results | Non-volatile matter content (%) | 65 | 65 | 65 | 65 | 65 | 65 | 65 | 65 | 65 | 65 | 60 | 65 | 65 | 65 | 65 | 60 |
| | Viscosity | DE | CD | E– | C | G– | F | E | I– | MN | JK | P | PQ | K– | V+ | U– | Q |
| | Gel ratio (%) | 99 | 99 | 99 | 99 | 99 | 98 | 98 | 99 | 97 | 98 | 97 | 99 | 98 | 99 | 98 | 98 |
| | Coating film hardness (Knoop hardness) | 10 | 10 | 11 | 8 | 12 | 13 | 14 | 11 | 13 | 14 | 8 | 9 | 13 | 10 | 12 | 6 |
| | Solvent resistance | O | O | O | O | O | O | O | O | O | O | O | O | O | O | O | O |

Respective comparisons of Example 36 with Comparative Example 6, Example 41 with Comparative Example 7, Example 43 with Comparative Example 8, Example 44 with Comparative Example 9 and Example 46 with Comparative Example 10 show that in all cases, the addition of the polyorthoesters allow the curable compositions to be reduced in a viscosity in the same non-volatile matter content and solvent composition without lowering the curing property and the hardness.

It can be found from the matters described above that the curable composition II according to the present invention can achieve a reduction in a viscosity without reducing the coating film performances by adding the polyorthoester and therefore is useful particularly as a binder for a high solid coating material.

Industrial Applicability

The polyorthoester of the present invention comprises an orthoester structure introduced into a hydroxyl group part of a hydroxyl group-containing compound and has a high degree of freedom in molecular design, and it can be applied to various fields and is industrially very useful. The polyorthoester of the present invention can be produced without causing gelation and a marked rise in a viscosity, and it has a low viscosity and can readily be controlled in a molecular weight. Further, the polyorthoester of the present invention can be produced by means of the same apparatus as a conventional polyester resin production apparatus in which alcohol produced by condensation can be distilled off, and therefore it does not require a specific apparatus and can readily be produced, so that it is industrially useful from such point of view.

The curable composition of the present invention is a composition comprising a polyorthoester and a curing agent. The polyorthoester comprises, as described above, an orthoester structure introduced into a hydroxyl group part of a hydroxyl group-containing compound and has a high degree of freedom in molecular design, and it can be applied to various fields and is industrially very useful. Further, the curable composition I of the present invention comprises a structure in which a hydroxyl group in a hydroxyl group-containing compound is blocked by the orthoester and therefore is free of problems originating in a hydroxyl group, for example, a problem on a rise in a viscosity of the curable composition, so that it can be increased in a solid content and is excellent in a compatibility, a wetting property to a base material and a storage stability.

## Claims

1. A process for the production of a polyorthoester **characterized by** subjecting, to condensation reaction,

(a) an orthoester represented by the following Formula (I):

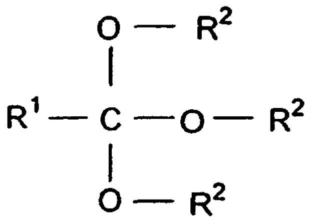

(I)

wherein $R^1$ represents a hydrogen atom or an alkyl group having 1 to 4 carbon atoms, and three $R^2$'s may be the same of different and each represent an alkyl group having 1 to 4 carbon atoms,
(b) at least one glycol compound selected from α-glycols and β-glycols, and
(c) a hydroxyl group-containing compound having at least two hydroxyl groups in a molecule other than the compound (b) as defined above.

2. The process of claim 1, wherein the orthoester (a) is at least one compound selected from the group consisting of methyl orthoformate, ethyl orthoformate, propyl orthoformate, butyl orthoformate, methyl orthoacetate, ethyl orthoacetate, methyl orthopropionate, ethyl orthopropionate, methyl orthobutyrate and ethyl orthobutyrate.

3. The process of claim 1, wherein the orthoester (a) is at least one compound selected from the group consisting of methyl orthoformate, ethyl orthoformate, methyl orthoacetate and ethyl orthoacetate.

4. The process of claim 1, wherein the glycol compound (b) is at least one glycol compound selected from the group

consisting of an α-glycol represented by the following Formula (II):

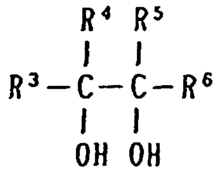

(II)

wherein $R^3$, $R^4$, $R^5$ and $R^6$ may be the same or different and each represent a hydrogen atom, an alkyl group having 1 to 24 carbon atoms, an aralkyl group having 7 to 24 carbon atoms or a phenyl group, or a group obtained by substituting a part of these groups with an oxygen atom, and the total of the carbon atoms in the groups represented by $R^3$, $R^4$, $R^5$ and $R^6$ falls in a range of 0 to 24; and $R^4$ and $R^5$ may form a cyclic structure together with carbon atoms to which they are bonded directly and
a β-glycol represented by the following Formula (III):

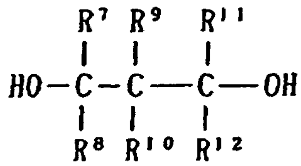

(III)

wherein $R^7$, $R^8$, $R^9$, $R^{10}$, $R^{11}$ and $R^{12}$ may be the same or different and each represent a hydrogen atom, an alkyl group having 1 to 24 carbon atoms, an aralkyl group having 7 to 24 carbon atoms or a phenyl group, or a group obtained by substituting a part of these groups with an oxygen atom, and the total of the carbon atoms in the groups represented by $R^7$, $R^8$, $R^9$, $R^{10}$, $R^{11}$ and $R^{12}$ falls in a range of 0 to 24; and $R^7$ and $R^9$ or $R^7$, $R^9$ and $R^{11}$ may form a cyclic structure together with carbon atoms to which they are bonded directly.

5. The process of claim 4, wherein the α-glycol is selected from the group consisting of ethylene glycol, 1,2-propylene glycol, 1,2-butylene glycol, 2,3-butylene glycol, 1,2-hexanediol, 1,2-dihydroxycyclohexane, pinacol, hydrolysis products of long chain alkyl monoepoxides, glycerin monoacetate (α product), glycerin monostearate (α product), 3-ethoxypropane-1,2-diol and 3-phenoxypropane-1,2-diol.

6. The process of claim 4, wherein the β-glycol is selected from the group consisting of neopentyl glycol, 2-methyl-1,3-propanediol, 2-methyl-2,4-pentanediol, 3-methyl-1,3-butanediol, 2-ethyl-1,3-hexanediol, 2,2-diethyl-1,3-propanediol, 2,2,4-trimethyl-1,3-pentanediol, 2-butyl-2-ethyl-1,3-propanediol, 2-phenoxypropane-1,3-diol, 2-methyl-2-phenylpropane-1,3-diol, 1,3-propylene glycol, 1,3-butylene glycol, dimethylolpropionic acid, dimethylolbutanoic acid, 2-ethyl-1,3-octanediol, 1,3-dihydroxycyclohexane, glycerin monoacetate (β product) and glycerin monostearate (β product).

7. The process of claim 1, wherein the glycol compound (b) is at least one compound selected from the group consisting of ethylene glycol, 1,2-propylene glycol, 1,2-hexanediol, neopentyl glycol, 2-methyl-1,3-propanediol, 2-methyl-2,4-pentanediol, 3-methyl-1,3-butanediol, 2-ethyl-1,3-hexanediol, 2,2-diethyl-1,3-propanediol, 2,2,4-trimethyl-1,3-pentanediol and 2-butyl-2-ethyl-1,3-propanediol.

8. The process of claim 1, wherein the hydroxyl group-containing compound (c) is at least one compound selected from the group consisting of 1,4-butanediol, 1,4-dihydroxycyclohexane, 1,5-pentanediol, 1,6-hexanediol, 2,5-hexanediol, 3-methyl-1,5-pentanediol, 1,4-dimethylolcyclohexane, tricyclodecanedimethanol, 2,2-dimethyl-3-hydroxypropyl-2,2-dimethyl-3-hydroxypropionate, bisphenol A, bisphenol F, bis(4-hydroxyhexyl)-2,2-propane, bis(4-hydroxyhexyl)methane, 3,9-bis(1,1-dimethyl-2-hydroxyethyl)-2,4,8,10-tetraoxaspiro[5,5]undecane, diethylene glycol, triethylene glycol, tetra- or more polyethylene glycol, dipropylene glycol, tripropylene glycol, tetra- or more polypropylene glycol, copolymers having hydroxyl groups at both terminals obtained by copolymerizing ethylene oxide with propylene oxide, polycaprolactonediol, polycarbonatediol, carboxylic acid adducts of diepoxide, glycerin, diglycerin, triglycerin, pentaerythritol, dipentaerythritol, sorbitol, mannit, trimethylolethane, trimethylolpropane, ditrimethylolpropane, tris(2-hydroxyethyl)isocyanurate, gluconic acid and polymers having 3 or more hydroxyl groups.

**9.** The process of claim 1, wherein the hydroxyl group-containing compound (c) has a molecular weight falling in a range of 90 to 100,000 and a hydroxyl group value falling in a range of 20 to 1,850 mg KOH/g.

**10.** The process of claim 1, wherein a hydroxyl group contained in the hydroxyl group-containing compound (c) is blocked by an orthoester of a 5-membered ring or a 6-membered ring constituted by the orthoester (a) and the glycol compound (b).

**11.** The polyorthoester having a structure represented by the following Formula (IV):

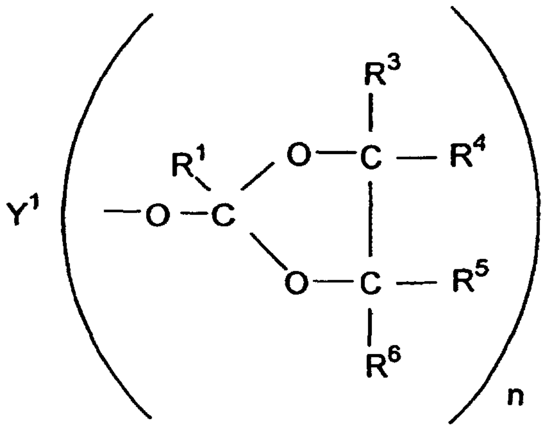

(IV)

wherein $Y^1$ represents a residue obtained by removing 3 to 40 hydroxyl groups from a compound having 3 to 40 hydroxyl groups in a molecule; $R^1$, $R^3$, $R^4$, $R^5$ and $R^6$ are the same as defined in claims 1 and 4; and n represents an integer of 3 to 40, or
the following Formula (V):

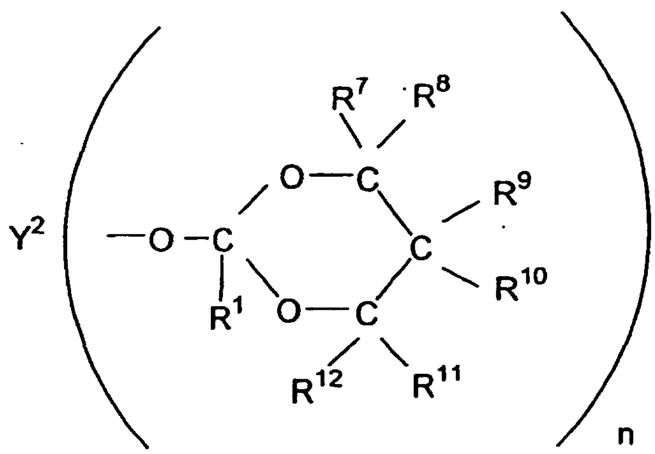

(V)

wherein $Y^2$ represents a residue obtained by removing 3 to 40 hydroxyl groups from a compound having 3 to 40 hydroxyl groups in a molecule; $R^1$, $R^7$, $R^8$, $R^9$, $R^{10}$, $R^{11}$ and $R^{12}$ are the same as defined in claims 1 and 4; and n represents an integer of 3 to 40.

**12.** The polyorthoester as set forth in claim 11 wherein $Y^1$ represents a tri- to hexavalent residue obtained by removing 3 to 6 hydroxyl groups from a compound having 3 to 6 hydroxyl groups in a molecule.

**13.** The polyorthoester as set forth in claim 11 wherein $Y^2$ represents a tri- to hexavalent residue obtained by removing 3 to 6 hydroxyl groups from a compound having 3 to 6 hydroxyl groups in a molecule."

**14.** A polyorthoester having a structure represented by the following Formula (VII):

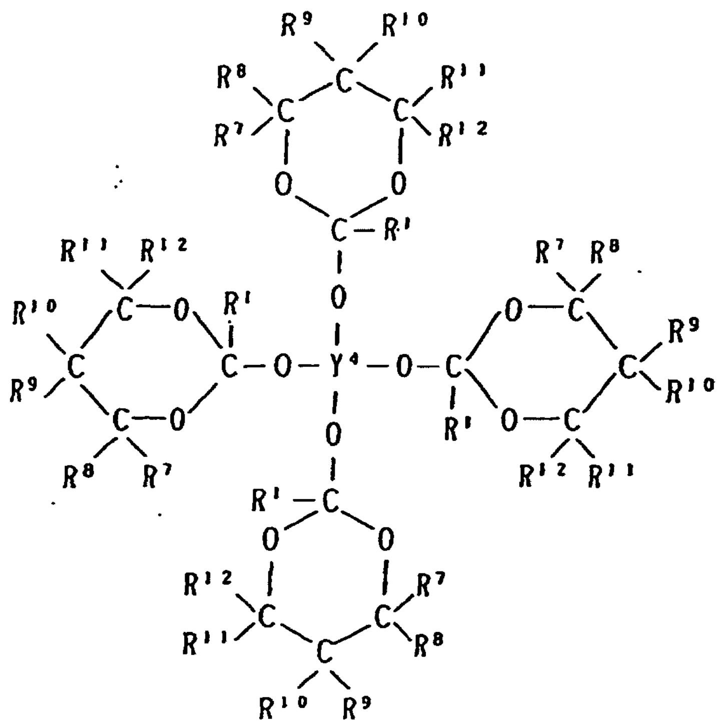

(VII)

wherein $Y^4$ represents a residue obtained by removing the following four hydroxyl groups from a compound having four hydroxyl groups in a molecule; and $R^1$, $R^7$, $R^8$, $R^9$, $R^{10}$, $R^{11}$ and $R^{12}$ are the same as defined in claims 1 and 4.

**15.** The polyorthoester as set forth in claim 11 or 14 which has been prepared by the process of any one of claims 1 to 10.

**16.** The process as set forth in claim 1, wherein the condensation reaction is carried out in the presence of an acid catalyst.

**17.** The process as described in claim 16, wherein the orthoester (a) in a proportion falling in a range of 0.05 to 5 moles is reacted with the glycol compound (b) in a proportion falling in a range of 0.05 to 5 moles each per equivalent of a hydroxyl group contained in the hydroxyl group-containing compound (c).

**18.** A curable composition comprising:

(A) the polyorthoester represented by the formula (IV) or (V) or (VII) as set forth in claim 11 or 14, and
(B) a curing agent having a group having a reactivity with a hydroxyl group.

**19.** The curable composition as described in claim 18, wherein the curing agent (B) is at least one compound or resin selected from the group consisting of polyisocyanate compounds, amino resins, epoxy group-containing compounds, alkoxysilyl group-containing compounds and compounds having two or more carboxylic anhydride groups.

**20.** The curable composition as described in claim 18, wherein the curing agent (B) is at least one compound or resin selected from the group consisting of polyisocyanate compounds and amino resins.

**21.** The curable composition as described in claim 18, comprising the polyorthoester (A) and the curing agent (B) in a range of 95/5 to 20/80 in terms of a solid matter weight ratio of (A)/(B).

**22.** The curable composition as described in claim 18, further comprising an acid catalyst.

**23.** The curable composition as described in claim 22, wherein the acid catalyst is at least one compound selected

from the group consisting of sulfonic acid compounds, neutralized compounds of the sulfonic acid compounds, organic phosphoric acid base compounds and neutralized compounds of the organic phosphoric acid base compounds.

**24.** The curable composition as described in claim 18, assuming the form of a coating material composition, an adhesive or an ink.

**25.** A curable composition comprising:

(1) a base polymer having a reactivity with the following curing agent (2),
(2) a curing agent which has a group having a reactivity with a hydroxyl group and which has a reactivity with the base polymer (1), and
(3) the polyorthoester represented by the formula (IV) or (V) or (VII) as set forth in claim 11 or 14.

**26.** The curable composition as described in claim 25, wherein the base polymer (1) is a hydroxyl group-containing polymer having a hydroxyl group value falling in a range of 20 to 300 mg KOH/g and a number average molecular weight falling in a range of 1,000 to 30,000.

**27.** The curable composition as described in claim 25, wherein the base polymer (1) is at least one polymer selected from the group consisting of hydroxyl group-containing acrylic resins and hydroxyl group-containing polyester resins.

**28.** The curable composition as described in claim 25, wherein the curing agent (2) is at least one compound selected from the group consisting of polyisocyanate compounds, amino resins, epoxy group-containing compounds, alkoxysilyl group-containing compounds and compounds having two or more carboxylic anhydride groups.

**29.** The curable composition as described in claim 25, wherein the curing agent (2) is at least one compound or resin selected from the group consisting of polyisocyanate compounds and amino resins.

**30.** The curable composition as described in claim 25, comprising the base polymer (1), the curing agent (2) and the polyorthoester (3) in a range of 20 to 89 parts by weight of the component (1), 5 to 70 parts by weight of the component (2) and 1 to 40 parts by weight of the component (3) based on 100 parts by weight of the total of solid matters contained in the respective components (1), (2) and (3).

**31.** The curable composition as described in claim 25, further comprising an acid catalyst.

**32.** The curable composition as described in claim 31, wherein the acid catalyst is at least one compound selected from the group consisting of sulfonic acid compounds, neutralized compounds of the sulfonic acid compounds, organic phosphoric acid base compounds and neutralized compounds of the organic phosphoric acid base compounds.

**33.** The curable composition as described in claim 25, assuming the form of a coating material composition, an adhesive or an ink.

**Patentansprüche**

**1.** Verfahren zur Herstellung eines Polyorthoesters, **dadurch gekennzeichnet, dass**

(a) ein Orthoester der nachstehenden allgemeinen Formel (I):

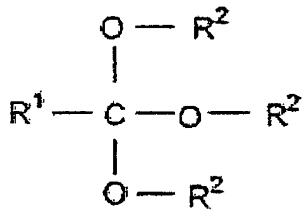

(I)

worin $R^1$ ein Wasserstoffatom oder eine Alkylgruppe mit 1 bis 4 Kohlenstoffatomen ist und die drei Reste $R^2$ gleich oder voneinander verschieden sind und jeweils eine Alkylgruppe mit 1 bis 4 Kohlenstoffatomen sind,
(b) mindestens eine Glykolverbindung ausgewählt aus α-Glykolen und β-Glykolen und
(c) eine Hydroxylgruppen-enthaltende Verbindung mit mindestens zwei Hydroxylgruppen im Molekül, die von der vorstehend definierten Verbindung (b) verschieden ist,
einer Kondensationsreaktion unterworfen werden.

2. Verfahren nach Anspruch 1, wobei der Orthoester (a) mindestens eine Verbindung, ausgewählt aus der Gruppe, bestehend aus Methylorthoformiat, Ethylorthoformiat, Propylorthoformiat, Butylorthoformiat, Methylorthoacetat, Ethylorthoacetat, Methylorthopropionat, Ethylorthopropionat, Methylorthobutyrat und Ethylorthobutyrat, ist.

3. Verfahren nach Anspruch 1, wobei der Orthoester (a) mindestens eine Verbindung, ausgewählt aus der Gruppe, bestehend aus Methylorthoformiat, Ethylorthoformiat, Methylorthoacetat und Ethylorthoacetat, ist.

4. Verfahren nach Anspruch 1, wobei die Glykolverbindung (b) mindestens eine Glykolverbindung ist, ausgewählt aus der Gruppe, bestehend aus einem α-Glykol der nachstehenden allgemeinen Formel (II):

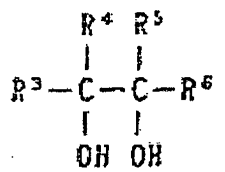

(II)

worin $R^3$, $R^4$, $R^5$ und $R^6$ gleich oder voneinander verschieden sein können und jeweils ein Wasserstoffatom, eine Alkylgruppe mit 1 bis 24 Kohlenstoffatomen, eine Aralkylgruppe mit 7 bis 24 Kohlenstoffatomen oder eine Phenylgruppe, oder eine Gruppe, erhalten durch Substitution eines Teils dieser Gruppen durch ein Sauerstoffatom, sind, und die Gesamtzahl der Kohlenstoffatome in den Gruppen $R^3$, $R^4$, $R^5$ und $R^6$ in einem Bereich von 0 bis 24 liegt, und $R^4$ und $R^5$ können zusammen mit den Kohlenstoffatomen, an die sie unmittelbar gebunden sind eine zyklische Struktur bilden, und
einem β-Glykol der nachstehenden allgemeinen Formel (III):

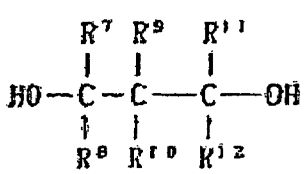

(III)

worin $R^7$, $R^8$, $R^9$, $R^{10}$, $R^{11}$ und $R^{12}$ gleich oder voneinander verschieden sein können und jeweils ein Wasserstoffatom, eine Alkylgruppe mit 1 bis 24 Kohlenstoffatomen, eine Aralkylgruppe mit 7 bis 24 Kohlenstoffatomen oder eine Phenylgruppe, oder eine Gruppe, erhalten durch Substitution eines Teils dieser Gruppen durch ein Sauerstoffatom, sind, und die Gesamtzahl der Kohlenstoffatome in den Gruppen $R^7$, $R^8$, $R^9$, $R^{10}$, $R^{11}$ und $R^{12}$ in einem Bereich von 0 bis 24 liegt, und $R^7$ und $R^9$ oder $R^7$, $R^9$ und $R^{11}$ können zusammen mit den Kohlenstoffatomen, an die sie unmittelbar gebunden, sind eine zyklische Struktur bilden.

5. Verfahren nach Anspruch 4, wobei das α-Glykol ausgewählt ist aus der Gruppe, bestehend aus Ethylenglykol,

1,2-Propylenglykol, 1,2-Butylenglykol, 2,3-Butylenglykol, 1,2-Hexandiol, 1,2-Dihydroxycyclohexan, Pinacol, Hydrolyseprodukten von langkettigen Alkylmonoepoxiden, Glycerinmonoacetat (α-Produkt), Glycerinmonostearat (α-Produkt), 3-Ethoxypropan-1,2-diol und 3-Phenoxypropan-1,2-diol.

**6.** Verfahren nach Anspruch 4, wobei das β-Glykol ausgewählt ist aus der Gruppe, bestehend aus Neopentylglykol, 2-Methyl-1,3-propandiol, 2-Methyl-2,4-pentandiol, 3-Methyl-1,3-butandiol, 2-Ethyl-1,3-hexandiol, 2,2-Diethyl-1,3-propandiol, 2,2,4-Trimethyl-1,3-pentandiol, 2-Butyl-2-ethyl-1,3-propandiol, 2-Phenoxypropan-1,3-diol, 2-Methyl-2-phenylpropan-1,3-diol, 1,3-Propylenglykol, 1,3-Butylenglykol, Dimethylolpropionsäure, Dimethylolbutansäure, 2-Ethyl-1,3-octandiol, 1,3-Dihydroxycyclohexan, Glycerinmonoacetat (β-Produkt) und Glycerinmonostearat (β-Produkt).

**7.** Verfahren nach Anspruch 1, wobei die Glykolverbindung (b) mindestens eine Verbindung ist, ausgewählt aus der Gruppe, bestehend aus Ethylenglykol, 1,2-Propylenglykol, 1,2-Hexandiol, Neopentylglykol, 2-Methyl-1,3-propandiol, 2-Methyl-2,4-pentandiol, 3-Methyl-1,3-butandiol, 2-Ethyl-1,3-hexandiol, 2,2-Diethyl-2,3-propandiol, 2,2,4-Trimethyl-1,3-pentandiol und 2-Butyl-2-ethyl-1,3-propandiol.

**8.** Verfahren nach Anspruch 1, wobei die Hydroxylgruppen-enthaltende Verbindung (c) mindestens eine Verbindung ist ausgewählt aus der Gruppe, bestehend aus 1,4-Butandiol, 1,4-Dihydroxycyclohexan, 1,5-Pentandiol, 1,6-Hexandiol, 2,5-Hexandiol, 3-Methyl-1,5-pentandiol, 1,4-Dimethylolcyclohexan, Tricyclodecandimezhanol, 2,2-Dimethyl-3-hydroxypropyl-2,2-dimethyl-3-hydroxypropionat, Bisphenol A, Bisphenol F, Bis(4-hydroxyhexyl)-2,2-propan, Bis(4-hydroxyhexyl)methan, 3,9-Bis(1,1-dimethyl-2-hydroxyethyl)-2,4,8,10-tetraoxaspiro[5,5]-undecan, Diethylenglykol, Triethylenglykol, Tetra- oder höheres Polyethylenglykol, Dipropylenglykol, Tripropylenglykol, Tetra- oder höheres Polypropylenglykol, Copolymer mit Hydroxylgruppen an beiden Enden, erhalten durch Copolymerisation von Ethylenoxid mit Propylenoxid, Polycaprolactondiol, Polycarbonatdiol, Carbonsäureaddukte, von Diepoxid, Glycerin, Diglycerin, Triglycerin, Pentaerythrit, Dipentaerythrit, Sorbit, Mannit, Trimethylolethan, Trimethylolpropan, Ditrimethylolpropan, Tris(2-hydroxyethyl)isocyanurat, Glukonsäure und Polymeren mit 3 oder mehr Hydroxylgruppen.

**9.** Verfahren nach Anspruch 1, wobei die Hydroxylgruppen-enthaltende Verbindung (c) ein Molekulargewicht aufweist in einem Bereich von 90 bis 100000 und eine Hydroxylzahl in einem Bereich von 20 bis 1850 mg KOH/g.

**10.** Verfahren nach Anspruch 1, wobei eine Hydroxylgruppe in der Hydroxylgruppen-enthaltenden Verbindung (c) blokkiert ist durch einen Orthoester eines 5-gliedrigen Rings oder eines 6-gliedrigen Rings, gebildet durch den Orthoester (a) und die Glykolverbindung (b).

**11.** Polyorthoester mit einer Struktur der nachstehenden allgemeinen Formel (IV):

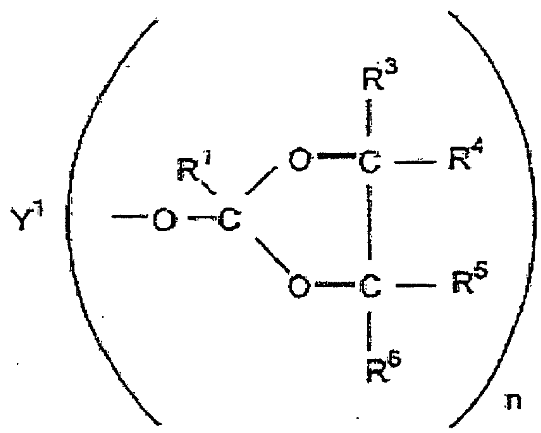

(IV)

worin $Y^1$ ein Rest ist, erhalten durch Entfernung von 3 bis 40 Hydroxylgruppen aus einer Verbindung mit 3 bis 40 Hydroxylgruppen im Molekül, $R^1$, $R^3$, $R^4$, $R^5$ und $R^6$ die in den Ansprüchen 1 und 4 angegebene Bedeutung haben, und n eine ganze Zahl von 3 bis 40 ist, oder
mit einer Struktur der nachstehenden allgemeinen Formel (V):

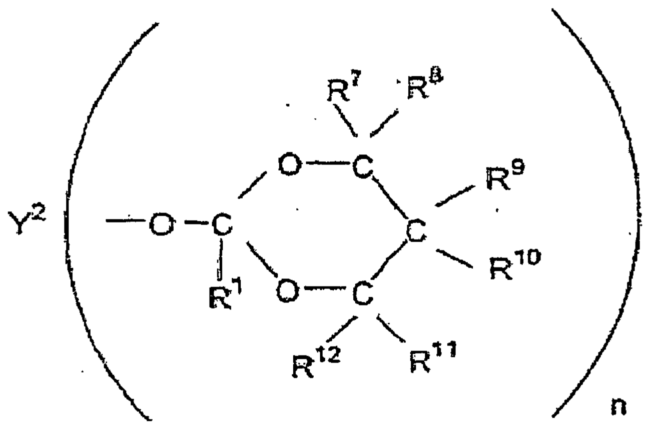

(V)

worin $Y^2$ ein Rest ist, erhalten durch Entfernung von 3 bis 40 Hydroxylgruppen aus einer Verbindung mit 3 bis 40 Hydroxylgruppen im Molekül, $R^1$, $R^7$, $R^8$, $R^9$, $R^{10}$, $R^{11}$ und $R^{12}$ die in den Ansprüchen 1 und 4 angegebene Bedeutung haben, und n eine ganze Zahl von 3 bis 40 ist.

**12.** Polyorthoester nach Anspruch 11, worin $Y^1$ ein 3- bis 6-wertiger Rest ist, erhalten durch Entfernung von 3 bis 6 Hydroxylgruppen aus einer Verbindung mit 3 bis 6 Hydroxylgruppen im Molekül.

**13.** Polyorthoester nach Anspruch 11, worin $Y^2$ ein 3- bis 6-wertiger Rest ist, erhalten durch Entfernung von 3 bis 6 Hydroxylgruppen aus einer Verbindung mit 3 bis 6 Hydroxylgruppen im Molekül.

**14.** Polyorthoester mit einer Struktur der nachstehenden allgemeinen Formel (VII):

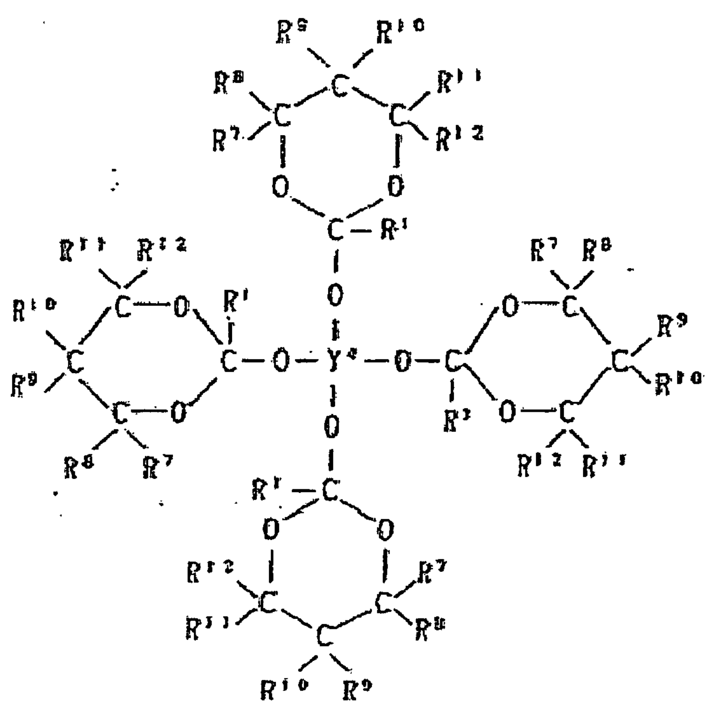

(VII)

worin $Y^4$ ein Rest ist, erhalten durch Entfernung der folgenden vier Hydroxylgruppen aus einer Verbindung mit vier Hydroxylgruppen im Molekül, und $R^1$, $R^7$, $R^8$, $R^9$, $R^{10}$, $R^{11}$ und $R^{12}$ die in den Ansprüchen 1 und 4 angegebene Bedeutung haben.

**15.** Polyorthoester nach Anspruch 11 oder 14, hergestellt nach dem Verfahren nach einem der Ansprüche 1 bis 10.

**16.** Verfahren nach Anspruch 1, wobei die Kondensationsreaktion in Gegenwart eines Säurekatalysators ausgeführt wird.

**17.** Verfahren nach Anspruch 16, wobei der Orthoester (a) in einem Anteil in einem Bereich von 0,05 bis 5 mol umgesetzt wird mit der Glykolverbindung (b) in einem Anteil in einem Bereich von 0,05 bis 5 mol jeweils pro Äquivalent einer Hydroxylgruppe, enthalten in der Hydroxylgruppen-enthaltenden Verbindung (c).

**18.** Härtbare Zusammensetzung, umfassend:

(A) den Polyorthoester der allgemeinen Formel (IV) oder (V) oder (VII) wie in Anspruch 11 oder 14 angegeben, und
(B) ein Härtungsmittel mit einer Gruppe, die mit einer Hydroxylgruppe reaktiv ist.

**19.** Härtbare Zusammensetzung nach Anspruch 18, wobei das Härtungsmittel (B) mindestens eine Verbindung oder ein Harz ist, ausgewählt aus der Gruppe, bestehend aus Polyisocyanatverbindungen, Aminoharzen, Epoxygruppen-enthaltenden Verbindungen, Alkoxysilylgruppen-enthaltenden Verbindungen und Verbindungen mit zwei oder mehreren Carbonsäureanhydridgruppen.

**20.** Härtbare Zusammensetzung nach Anspruch 18, wobei das Härtungsmittel (B) mindestens eine Verbindung oder ein Harz ist, ausgewählt aus der Gruppe, bestehend aus Polyisocyanatverbindungen und Aminoharzen.

**21.** Härtbare Zusammensetzung nach Anspruch 18, umfassend den Polyorthoester (A) und das Härtungsmittel (B) in einem Bereich von 95/5 bis 20/80 des Feststoffgewichtsverhältnisses von (A)/(B).

**22.** Härtbare Zusammensetzung nach Anspruch 18, die des Weiteren einen Säurekatalysator umfasst.

**23.** Härtbare Zusammensetzung nach Anspruch 22, wobei der Säurekatalysator mindestens eine Verbindung ist ausgewählt aus der Gruppe, bestehend aus Sulfonsäureverbindungen, neutralisierten Verbindungen der Sulfonsäureverbindungen, organischen Phosphorsäuregrundverbindungen und neutralisierten Verbindungen der organischen Phosphorsäuregrundverbindungen.

**24.** Härtbare Zusammensetzung nach Anspruch 18 in Form einer Beschichtungsmaterialzusammensetzung, eines Klebstoffs oder einer Druckfarbe bzw. Tinte.

**25.** Härtbare Zusammensetzung, umfassend:

(1) ein Grundpolymer, das reaktiv ist mit dem nachstehenden Härtungsmittel (2),
(2) ein Härtungsmittel mit einer Gruppe, die reaktiv ist mit einer Hydroxylgruppe und die reaktiv ist mit dem Grundpolymer (1), und
(3) den Polyorthoester der allgemeinen Formel (IV) oder (V) oder (VII) wie in Anspruch 11 oder 14 angegeben.

**26.** Härtbare Zusammensetzung nach Anspruch 25, wobei das Grundpolymer (1) ein Hydroxylgruppen-enthaltendes Polymer mit einer Hydroxylzahl in einem Bereich von 20 bis 300 mg KOH/g und einem zahlenmittleren Molekulargewicht in einem Bereich von 1000 bis 30000 ist.

**27.** Härtbare Zusammensetzung nach Anspruch 25, wobei das Grundpolymer (1) mindestens ein Polymer ist, ausgewählt aus der Gruppe, bestehend aus Hydroxylgruppen-enthaltenden Acrylharzen und Hydroxylgruppen-enthaltenden Polyesterharzen.

**28.** Härtbare Zusammensetzung nach Anspruch 25, wobei das Härtungsmittel (2) mindestens eine Verbindung ist, ausgewählt aus der Gruppe, bestehend aus Polyisocyanatverbindungen, Aminoharzen, Epoxygruppen-enthaltenden Verbindungen, Alkoxysilylgruppen-enthaltenden Verbindungen und Verbindungen mit 2 oder mehreren Carbonsäureanhydridgruppen.

**29.** Härtbare Zusammensetzung nach Anspruch 25, wobei das Härtungsmittel (2) mindestens eine Verbindung oder ein Harz ist, ausgewählt aus der Gruppe, bestehend aus Polyisocyanatverbindungen und Aminoharzen.

**30.** Härtbare Zusammensetzung nach Anspruch 25, umfassend das Grundpolymer (1), das Härtungsmittel (2) und

den Polyorthoester (3) in einem Bereich von 20 bis 89 Gewichtsteilen der Komponente (1), 5 bis 70 Gewichtsteilen der Komponente (2) und 1 bis 40 Gewichtsteilen der Komponente (3) bezogen auf 100 Gewichtsteile des in den entsprechenden Komponenten (1), (2) und (3) enthaltenen Gesamtfeststoffs.

**31.** Härtbare Zusammensetzung nach Anspruch 25, die des Weiteren einen Säurekatalysator umfasst.

**32.** Härtbare Zusammensetzung nach Anspruch 31, wobei der Säurekatalysator mindestens eine Verbindung ist, ausgewählt aus der Gruppe, bestehend aus Sulfonsäureverbindungen, neutralisierten Verbindungen der Sulfonsäureverbindungen, organischen Phosphorsäuregrundverbindungen und neutralisierten Verbindungen der organischen Phosphorsäuregrundverbindungen.

**33.** Härtbare Zusammensetzung nach Anspruch 25 in Form einer Beschichtungsmaterialzusammensetzung, eines Klebstoffs oder einer Druckfarbe bzw. Tinte.

**Revendications**

**1.** Procédé pour la production d'un polyorthoester **caractérisé par** la soumission à une réaction de condensation

(a) d'un orthoester représenté par la Formule (I) suivante ;

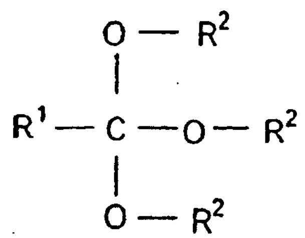

(I)

dans laquelle $R^1$ représente un atome d'hydrogène ou un groupe alkyle ayant 1 à 4 atomes de carbone, et trois $R^2$ qui peuvent être identiques ou différents et représentent chacun un groupe alkyle ayant 1 à 4 atomes de carbone,

(b) au moins d'un composé glycol choisi parmi des α-glycols et des β-glycols, et

(c) d'un composé contenant des groupes hydroxyle, ayant au moins deux groupes hydroxyle dans une molécule, autre que le composé (b) défini ci-dessus.

**2.** Procédé selon la revendication 1, dans lequel l'orthoester (a) est au moins un composé choisi au sein du groupe consistant en de l'orthoformiate de méthyle, de l'orthoformiate d'éthyle, de l'orthoformiate de propyle, de l'orthoformiate de butyle, de l'orthoacétate de méthyle, de l'orthoacétate d'éthyle, de l'orthopropionate de méthyle, de l'orthopropionate d'éthyle, de l'orthobutyrate de méthyle et de l'orthobutyrate d'éthyle.

**3.** Procédé selon la revendication 1, dans lequel l'orthoester (a) est au moins un composé choisi au sein du groupe consistant en de l'orthoformiate de méthyle, de l'orthoformiate d'éthyle, de l'orthoacétate de méthyle et de l'orthoacétate d'éthyle.

**4.** Procédé selon la revendication 1, dans lequel le composé glycol (b) est au moins un composé glycol choisi au sein du groupe consistant en un α-glycol représenté par la Formule (II) suivante :

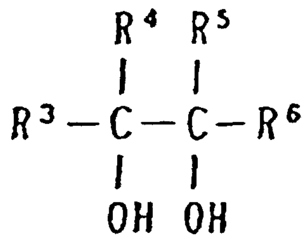

(II)

dans laquelle $R^3$, $R^4$, $R^5$ et $R^6$ peuvent être identiques ou différents et représentent chacun un atome d'hydrogène, un groupe alkyle ayant 1 à 24 atomes de carbone, un groupe aralkyle ayant 7 à 24 atomes de carbone ou un groupe phényle, ou un groupe obtenu par substitution d'une partie de ces groupes par un atome d'oxygène, et le nombre total des atomes de carbone dans les groupes représentés par $R^3$, $R^4$, $R^5$ et $R^6$ se situe dans l'intervalle de 0 à 24 ; et $R^4$ et $R^5$ peuvent former une structure cyclique en association avec les atomes de carbone auxquels ils sont directement liés, et
un β-glycol représenté par la Formule (III) suivante :

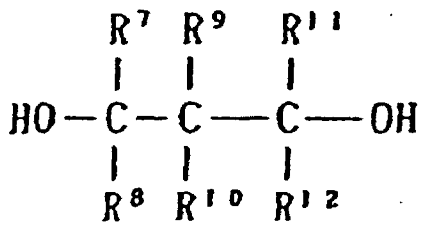

(III)

dans laquelle $R^7$, $R^8$, $R^9$, $R^{10}$, $R^{11}$ et $R^{12}$ peuvent être identiques ou différents et représentent chacun un atome d'hydrogène, un groupe alkyle ayant 1 à 24 atomes de carbone, un groupe aralkyle ayant 7 à 24 atomes de carbone ou un groupe phényle, ou un groupe obtenu par substitution d'une partie de ces groupes par un atome d'oxygène, et le nombre total des atomes de carbone dans les groupes représentés par $R^7$, $R^8$, $R^9$, $R^{10}$, $R^{11}$ et $R^{12}$ se situe dans l'intervalle de 0 à 24 ; et $R^7$ et $R^9$, ou $R^7$, $R^9$, et $R^{11}$ peuvent former une structure cyclique en association avec les atomes de carbone auxquels ils sont directement liés.

**5.** Procédé selon la revendication 4, dans lequel l'α-glycol est choisi au sein du groupe consistant en de l'éthylèneglycol, du 1,2-propylèneglycol, du 1,2-butylèneglycol, du 2,3-butylèneglycol, de 1,2-hexanediol, du 1,2-dihydroxycyclohexane, du pinacol, des produits d'hydrolyse d'alkylmonoépoxydes à longue chaîne, du monoacétate de glycérol (produit α), du monostéarate de glycérol (produit α), du 3-éthoxypropane-1,2-diol et du 3-phénoxypropane-1,2-diol.

**6.** Procédé selon la revendication 4, dans lequel le β-glycol est choisi au sein du groupe consistant en du néopentylglycol, du 2-méthyl-1,3-propanediol, du 2-méthyl-2,4-pentanediol, du 3-méthyl-1,3-butanediol, du 2-éthyl-1,3-hexanediol, du 2,2-diéthyl-1,3-propanediol, du 2,2,4-triméthyl-1,3-pentanediol, du 2-butyl-2-éthyl-1,3-propanediol, du 2-phénoxypropane-1,3-diol, du 2-méthyl-2-phénylpropane-1,3-diol, du 1,3-propylèneglycol, du 1,3-butylèneglycol, de l'acide diméthylolpropionique, de l'acide diméthylolbutanoïque, du 2-éthyl-1,3-octanediol, du 1,3-dihydroxycyclohexane, du monoacétate de glycérol (produit β) et du monostéarate de glycérol (produit β),

**7.** Procédé selon la revendication 1, dans lequel le composé glycol (b) est au moins un composé choisi au sein du groupe consistant en de l'éthylèneglycol, du 1,2-propylèneglycol, du 1,2-hexanediol, du néopentylglycol, du 2-méthyl-1,3-propanediol, du 2-méthyl-2,4-pentanediol, du 3-méthyl-1,3-butanediol, du 2-éthyl-1,3-hexanediol, du 2,2-diéthyl-1,3-propanediol, du 2,2,4-triméthyl-1,3-pentanediol et du 2-butyl-2-éthyl- 1,3-propanediol.

**8.** Procédé selon la revendication 1, dans lequel le composé (c) contenant des groupes hydroxyle est au moins un composé choisi au sein du groupe consistant en du 1,4-butanediol, du 1,4-dihydroxycyclohexane, du 1,5-pentanediol, du 1,6-hexanediol, du 2,5-hexanediol, du 3-méthyl-1,5-pentanediol, du 1,4-diméthylolcyclohexane, du tricyclodécanediméthanol, du 2,2-diméthyl-3-hydroxypropyl-2,2-diméthyl-3-hydroxypropionate, du bisphénol A, du bisphénol F, du bis(4-hydroxyhexyl)-2,2-propane, du bis(4-hydroxyhexyl)méthane, du 3,9-bis(1,1-diméthyl-2-hydroxyéthyl)-2,4,8,10-tétraoxaspiro[5,5]undécane, du diéthylèneglycol, du triéthylèneglycol, du tétra- (ou plus)-polyéthylène-glycol, du di propylèneglycol, du tripropylèneglycol, du tétra- (ou plus)-polypropylèneglycol, des copolymères ayant des groupes hydroxyle aux deux extrémités terminales, obtenus par copolymérisation de l'oxyde d'éthylène avec l'oxyde de propylène, du polycaprolactonediol, du polycarbonatediol, des adduits d'acides carboxyliques de diépoxyde, du glycérol, du diglycérol, du triglycérol, du pentaérythritol, du dipentaérythritol, du sorbitol, de la mannite, du triméthyloléthane, du triméthylolpropane, du ditriméthylolpropane, du tris(2-hydroxyéthyl) isocyanurate, de l'acide gluconique et de polymères ayant 3 groupes hydroxyle ou plus.

**9.** Procédé selon la revendication 1, dans lequel le composé (c) contenant des groupes hydroxyle a un poids moléculaire se situant dans l'intervalle de 90 à 100.000 et un indice d'hydroxyle se situant dans l'intervalle de 20 à 1.850 mg de KOH/g.

**10.** Procédé selon la revendication 1, dans lequel un groupe hydroxyle contenu dans le composé (c) contenant des groupes hydroxyle est bloqué par un orthoester d'un cycle à 5 atomes ou à 6 atomes constitué par l'orthoester (a) et le composé glycol (b).

**11.** Polyorthoester ayant une structure représentée par la Formule (IV) suivante :

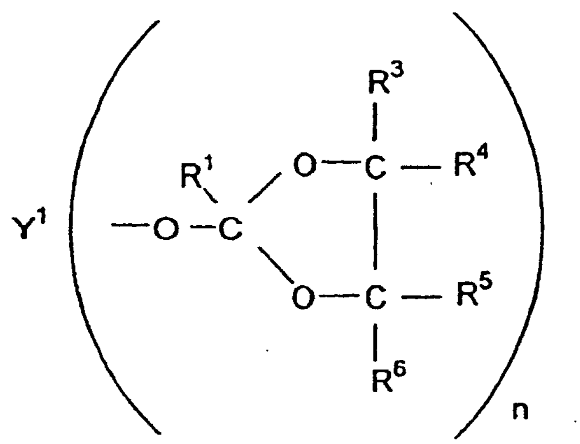

(IV)

dans laquelle $Y^1$ représente un résidu obtenu par élimination de 3 à 40 groupes hydroxyle à partir d'un composé ayant 3 à 40 groupes hydroxyle dans une molécule ; $R^1$, $R^3$, $R^4$, $R^5$ et $R^6$ sont tels que définis dans les revendications 1 et 4 ; et n représente un nombre entier de 3 à 40, ou bien par la Formule (V) suivante :

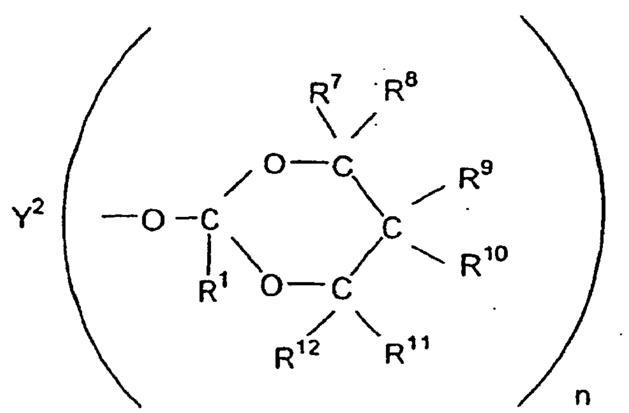

(V)

dans laquelle $Y^2$ représente un résidu obtenu par élimination de 3 à 40 groupes hydroxyle à partir d'un composé

ayant 3 à 40 groupes hydroxyle dans une molécule ; $R^1$, $R^7$, $R^8$, $R^9$, $R^{10}$, $R^{11}$ et $R^{12}$ sont tels que définis dans les revendications 1 et 4 ; et n représente un nombre entier de 3 à 40.

**12.** Polyorthoester selon la revendication 11, dans lequel $Y^1$ représente un résidu tri- à hexa-valent obtenu par élimination de 3 à 6 groupes hydroxyle à partir d'un composé ayant 3 à 6 groupes hydroxyle dans une molécule.

**13.** Polyorthoester selon la revendication 11, dans lequel $Y^2$ représente un résidu tri- à hexa-valent obtenu par élimination de 3 à 6 groupes hydroxyle à partir d'un composé ayant 3 à 6 groupes hydroxyle dans une molécule.

**14.** Polyorthoester ayant une structure représentée par la Formule (VII) suivante :

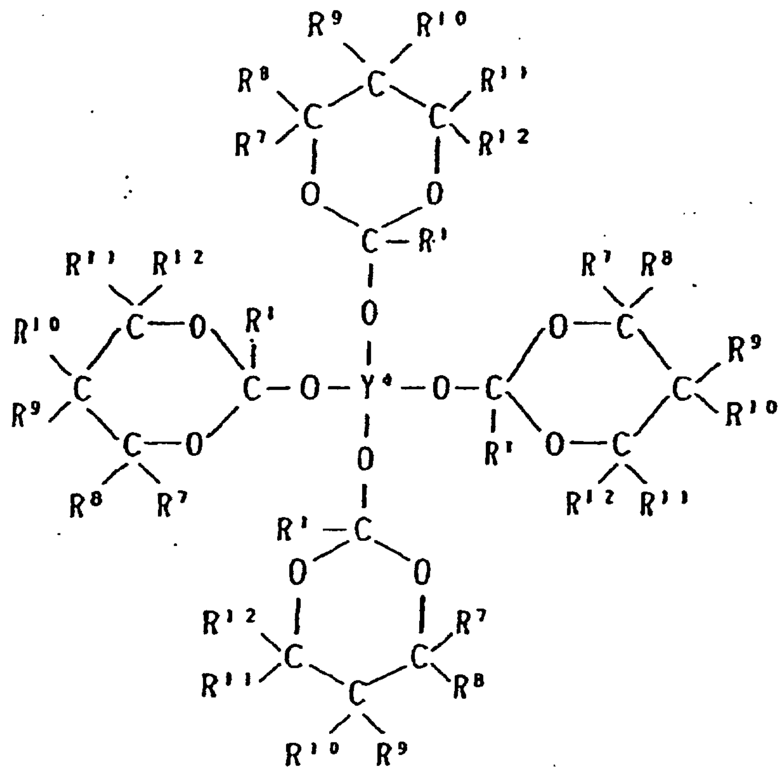

(VII)

dans laquelle $Y^4$ représente un résidu obtenu par l'élimination de quatre groupes hydroxyle suivants à partir d'un composé ayant quatre groupe hydroxyle dans une molécule ; et $R^1$, $R^7$, $R^8$, $R^9$, $R^{10}$, $R^{11}$ et $R^{12}$ sont tels que définis dans les revendications 1 et 4.

**15.** Polyorthoester selon la revendication 11 ou 14, qui a été préparé par le procédé selon l'une quelconque des revendications 1 à 10.

**16.** Procédé selon la revendication 1, dans lequel la réaction de condensation est effectuée en présence d'un catalyseur acide.

**17.** Procédé selon la revendication 16, dans lequel l'orthoester (a) dans une proportion se situant dans l'intervalle de 0,05 à 5 moles est mis en réaction avec le composé glycol (b) dans une proportion comprise dans l'intervalle de 0,05 à 5 moles, chacun par équivalent d'un groupe hydroxyle contenu dans le composé (c) contenant des groupes hydroxyle.

**18.** Composition durcissable comprenant :

(A) le polyorthoester représenté par la Formule (IV) ou (V) ou (VII) selon la revendication 11 ou 14, et
(B) un agent durcisseur ayant un groupe possédant une réactivité vis-à-vis d'un groupe hydroxyle.

**19.** Composition durcissable selon la revendication 18, dans laquelle l'agent durcisseur (B) est au moins un composé

ou une résine choisi au sein du groupe consistant en des composés polyisocyanate, des résines amino, des composés contenant des groupes époxy, des composés contenant des groupes alcoxysilyle et des composés ayant deux ou plusieurs groupes anhydride carboxylique.

**20.** Composition durcissable selon la revendication 18, dans laquelle l'agent durcisseur (B) est au moins un composé ou une résine choisi au sein du groupe consistant en des composés polyisocyanate et en des résines amino.

**21.** Composition durcissable selon la revendication 18, comprenant le polyorthoester (A) et l'agent durcisseur (B) dans l'intervalle de 95/5 à 20/80 exprimées en termes de rapport pondéral des matières solides (A)/(B).

**22.** Composition durcissable selon la revendication 18, comprenant également un catalyseur acide.

**23.** Composition durcissable selon la revendication 22, dans laquelle le catalyseur acide est au moins un composé choisi au sein du groupe consistant en des composés acide sulfonique, des composés neutralisés des composés acide sulfonique, des composés organiques à base d'acide phosphorique et des composés neutralisés des composés organiques à base d'acide phosphorique.

**24.** Composition durcissable selon la revendication 18, se présentant sous la forme d'une composition de matériau de revêtement, d'un adhésif ou d'une encre.

**25.** Composition durcissable comprenant :

- (1) une base polymère ayant une réactivité vis-à-vis de l'agent durcisseur (2) suivant,
- (2) un agent durcisseur qui a un groupe ayant une réactivité vis-à-vis d'un groupe hydroxyle et qui a une réactivité vis-à-vis de la base polymère (1), et
- (3) le polyorthoester représenté par les Formules (IV) ou (V) ou (VII) selon la revendication 11 ou 14.

**26.** Composition durcissable selon la revendication 25, dans laquelle la base polymère (1) est un polymère contenant des groupes hydroxyle, ayant un indice d'hydroxyle se situant dans l'intervalle de 20 à 300 mg KOH/g et un poids moléculaire moyen en nombre se situant dans l'intervalle de 1.000 à 30.000.

**27.** Composition durcissable selon la revendication 25, dans laquelle la base polymère (1) est au moins un polymère choisi au sein du groupe consistant en des résines acryliques contenant des groupes hydroxyle, et des résines polyester contenant des groupes hydroxyle.

**28.** Composition durcissable selon la revendication 25, dans laquelle l'agent durcisseur (2) est au moins un composé choisi au sein du groupe consistant en des composés polyisocyanate, des résines amino, des composés contenant des groupes époxy, des composés contenant des groupes alcoxysilyle et des composés ayant deux ou plusieurs groupes anhydride carboxylique.

**29.** Composition durcissable selon la revendication 25, dans laquelle l'agent durcisseur (2) est au moins un composé ou une résine choisie au sein du groupe consistant en des composés polyisocyanate et en des résines amino.

**30.** Composition durcissable selon la revendication 25, comprenant la base polymère (1), l'agent durcisseur (2) et le polyorthoester (3) à raison de 20 à 89 parties en poids du composant (1), 5 à 70 parties en poids du composant (2) et 1 à 40 parties en poids du composant (3) par rapport à 100 parties en poids des matières solides totales contenues dans les composants respectifs (I), (2) et (3).

**31.** Composition durcissable selon la revendication 25, comprenant aussi un catalyseur acide.

**32.** Composition durcissable selon la revendication 31, dans laquelle le catalyseur acide est au moins un composé choisi au sein du groupe consistant en des composés acide sulfonique, des composés neutralisés des composés acide sulfonique, des composés organiques à base d'acide phosphorique et des composés neutralisés des composés organiques à base d'acide phosphorique.

**33.** Composition durcissable selon la revendication 25, se présentant sous la forme d'une composition de matériau de revêtement, d'un adhésif ou d'une encre.